# EUROPEAN PATENT SPECIFICATION

(11) **EP 2 529 698 B1**
(45) Date of publication and mention of the grant of the patent: **29.01.2014**
(21) Application number: 12179338.4
(22) Date of filing: 22.12.2004
(51) Int. Cl.: A61F 2/01, A61F 2/24

(54) **Repositionable heart valve**
Umpositionierbare Herzklappe
Valvule cardiaque repositionnable

(30) Priority: 23.12.2003 US 746280; 23.12.2003 US 746942; 23.12.2003 US 746240; 23.12.2003 US 746872; 23.12.2003 US 746887; 23.12.2003 US 746120; 23.12.2003 US 746285; 15.07.2004 US 893151; 15.07.2004 US 893131; 15.07.2004 US 893143; 15.07.2004 US 893142; 21.10.2004 US 972287; 21.10.2004 US 971535; 05.11.2004 US 982692; 05.11.2004 US 982388
(43) Date of publication of application: 05.12.2012
(62) Divisional of application: 04815634.3
(73) Proprietor: Sadra Medical, Inc., Campbell, CA 95008 (US)
(72) Inventor: Salahieh, Amr, Saratoga, CA California 95070 (US); Brandt, Brian, D., San Jose, CA California 95119 (US); Morejohn, Dwight, P., Davis, CA California 95616 (US); Haug, Ulrich, R., Campbell, CA California 95008 (US); Dueri, Jean-Pierre, Stockton, CA California 95219 (US); Valencia, Hans, F., San Jose, CA California 95125 (US); Geshlider, Robert, A., San Francisco, CA California 94131 (US); Krolik, Jeff, Campbell, CA California 95008 (US); Saul, Tom, Moss Beach, CA California 94038 (US); Argento, Claudio, Los Gatos, CA California 95033 (US); Hildebrand, Daniel, Menlo Park, CA California 94025 (US)
(74) Representative: Peterreins, Frank

(56) References cited:
- US-A- 5 957 949
- US-A1- 2002 123 802

## Description

### BACKGROUND OF THE INVENTION

The present invention relates to methods and apparatus for endovascularly replacing a heart valve. More particularly, the present invention relates to methods and apparatus for percutaneously replacing a heart valve with a replacement valve using an expandable and retrievable anchor.

Heart valve surgery is used to repair or replace diseased heart valves. Valve surgery is an open-heart procedure conducted under general anesthesia. An incision is made through the patient's sternum (sternotomy), and the patient's heart is stopped while blood flow is rerouted through a heart-lung bypass machine.

Valve replacement may be indicated when there is a narrowing of the native heart valve, commonly referred to as stenosis, or when the native valve leaks or regurgitates.

When replacing the valve, the native valve is excised and replaced with either a biologic or a mechanical valve. Mechanical valves require lifelong anticoagulant medication to prevent blood clot formation, and clicking of the valve often may be heard through the chest. Biologic tissue valves typically do not require such medication. Tissue valves may be obtained from cadavers or may be porcine or bovine, and are commonly attached to synthetic rings that are secured to the patient's heart.

Valve replacement surgery is a highly invasive operation with significant concomitant risk. Risks include bleeding, infection, stroke, heart attack, arrhythmia, renal failure, adverse reactions to the anesthesia medications, as well as sudden death. 2-5% of patients die during surgery.

Post-surgery, patients temporarily may be confused due to emboli and other factors associated with the heart-lung machine. The first 2-3 days following surgery are spent in an intensive care unit where heart functions can be closely monitored. The average hospital stay is between 1 to 2 weeks, with several more weeks to months required for complete recovery.

In recent years, advancements in minimally invasive surgery and interventional cardiology have encouraged some investigators to pursue percutaneous replacement of the aortic heart valve. Percutaneous Valve Technologies ("PVT") of Fort Lee, New Jersey, has developed a balloon-expandable stent integrated with a bioprosthetic valve. The stent/valve device is deployed across the native diseased valve to permanently hold the valve open, thereby alleviating a need to excise the native valve and to position the bioprosthetic valve in place of the native valve. PVT's device is designed for delivery in a cardiac catheterization laboratory under local anesthesia using fluoroscopic guidance, thereby avoiding general anesthesia and open-heart surgery. The device was first implanted in a patient in April of 2002.

PVT's device suffers from several drawbacks. Deployment of PVT's stent is not reversible, and the stent is not retrievable. This is a critical drawback because improper positioning too far up towards the aorta risks blocking the coronary ostia of the patient. Furthermore, a misplaced stent/valve in the other direction (away from the aorta, closer to the ventricle) will impinge on the mitral apparatus and eventually wear through the leaflet as the leaflet continuously rubs against the edge of the stent/valve.

Another drawback of the PVT device is its relatively large cross-sectional delivery profile. The PVT system's stent/valve combination is mounted onto a delivery balloon, making retrograde delivery through the aorta challenging. An antegrade transseptal approach may therefore be needed, requiring puncture of the septum and routing through the mitral valve, which significantly increases complexity and risk of the procedure. Very few cardiologists are currently trained in performing a transseptal puncture, which is a challenging procedure by itself.

Other prior art replacement heart valves use self-expanding stents as anchors. In the endovascular aortic valve replacement procedure, accurate placement of aortic valves relative to coronary ostia and the mitral valve is critical. Standard self-expanding systems have very poor accuracy in deployment, however. Often the proximal end of the stent is not released from the delivery system until accurate placement is verified by fluoroscopy, and the stent typically jumps once released. It is therefore often impossible to know where the ends of the stent will be with respect to the native valve, the coronary ostia and the mitral valve.

Also, visualization of the way the new valve is functioning prior to final deployment is very desirable. Visualization prior to final and irreversible deployment cannot be done with standard self-expanding systems, however, and the replacement valve is often not fully functional before final deployment.

Another drawback of prior art self-expanding replacement heart valve systems is their lack of radial strength. In order for self-expanding systems to be easily delivered through a delivery sheath, the metal needs to flex and bend inside the delivery catheter without being plastically deformed. In arterial stents, this is not a challenge, and there are many commercial arterial stent systems that apply adequate radial force against the vessel wall and yet can collapse to a small enough of a diameter to fit inside a delivery catheter without plastically deforming.

However when the stent has a valve fastened inside it, as is the case in aortic valve replacement, the anchoring of the stent to vessel walls is significantly challenged during diastole. The force to hold back arterial pressure and prevent blood from going back inside the ventricle during diastole will be directly transferred to the stent/vessel wall interface. Therefore the amount of radial force required to keep the self expanding stent/valve in contact with the vessel wall and not sliding will be much higher than in stents that do not have valves inside of them. Moreover, a self-expanding stent without sufficient radial force will end up dilating and contracting with each heartbeat, thereby distorting the valve, affecting its function and possibly migrating and dislodging completely. Simply increasing strut thickness of the self-expanding stent is not a practical solution as it runs the risk of larger profile and/or plastic deformation of the self-expanding stent.

U.S. patent application Serial No. 2002/0151970 to Garrison et al. describes a two-piece device for replacement of the aortic valve that is adapted for delivery through a patient's aorta. A stent is percutaneously placed across the native valve, then a replacement valve is positioned within the lumen of the stent. By separating the stent and the valve during delivery, a profile of the device's delivery system may be sufficiently reduced to allow aortic delivery without requiring a transseptal approach. Both the stent and a frame of the replacement valve may be balloon-expandable or self-expanding.

While providing for an aortic approach, devices described in the Garrison patent application suffer from several drawbacks. First, the stent portion of the device is delivered across the native valve as a single piece in a single step, which precludes dynamic repositioning of the stent during delivery. Stent foreshortening or migration during expansion may lead to improper alignment.

Additionally, Garrison's stent simply crushes the native valve leaflets against the heart wall and does not engage the leaflets in a manner that would provide positive registration of the device relative to the native position of the valve. This increases an immediate risk of blocking the coronary ostia, as well as a longer-term risk of migration of the device post-implantation. FurtherstiU, the stent comprises openings or gaps in which the replacement valve is seated post-delivery. Tissue may protrude through these gaps, thereby increasing a risk of improper seating of the valve within the stent.

US 5,957,949 discloses an artificial valve including a tubular graft having radially compressible annular spring portions for biasing proximal and distal ends of the graft into conforming fixed engagement with the interior surface of a generally tubular passage. Also disclosed is a deployment catheter including an inner catheter having a nitinol core wire, a controllable tip balloon at its distal end for dilatation and occlusion of the passage.

### SUMMARY OF THE INVENTION

The object of the present invention is to provide an apparatus and a replacement valve, wherein the replacement valve can be secured safely at the implantation site, and which enables a repositioning of the anchor after an initial positioning.

This object is solved by an apparatus according to claim 1. The dependent claims depict advantageous features of the present invention.

### BRIEF DESCRIPTION OF THE DRAWINGS

Figures 1A and 1B show replacement valve apparatus in accordance with the present invention. Figure 1A illustrates the apparatus in a collapsed delivery configuration within a delivery system. Figure 1B illustrates the apparatus in an expanded configuration partially deployed from the delivery system.
Figures 2A-F show an anchor of the apparatus of Figures 1 in the collapsed delivery configuration and the expanded deployed configuration, as well as the full apparatus in the deployed configuration, and optional locking mechanisms for use with the apparatus.
Figure 3 shows a detail view of a variation of an anchor post.
Figures 4A and 4B show an alternative variation of the post having a lock alignment feature.
Figures 5A and 5B show a variation of the post having an alternative lock alignment feature.
Figure 6 shows a variation of the post having an expansile element.
Figure 7 shows a variation of the post with an alternative expansile or cable element.
Figures 8A-C show a variation of the post having an alternative lock alignment feature.
Figure 9 shows the post variation of Figure 3 in combination with an illustrative actuator and release actuator.
Figures 10A-C show a variation of the post, actuator and release actuator that form an alternative releasable attachment mechanism.
Figures 11A-C show another variation of the releasable attachment mechanism.
Figures 12A-C show yet another variation of the releasable attachment mechanism.
Figures 13A and 13B show still another variation of the releasable attachment element.
Figure 14 shows a variation of the post, actuator and anchor lock element having a reversible lock.
Figures 15A-C show a variation of the actuator, lock actuator and release actuator.
Figure 16 shows a variation of the anchor lock element having a lock alignment feature.
Figures 17A and 17B show expansion, locking and actuation of the releasable attachment mechanism of the apparatus of Figure 16.
Figure 18 shows another variation of the apparatus having an actuable lock prevention mechanism.
Figures 19A and 19B show a variation of the post that is configured to lock against the braid of the anchor.
Figures 20A-C show actuation and release of a variation of the anchor lock element.
Figures 21A and 21B show another variation of a releasable actuation mechanism having a lock alignment mechanism which can be cut from a tube.
Figures 22A-D show actuation of a variation of the anchor lock element that may be formed from a cut tube.
Figures 23A-23F show a variation of the post having an unlock actuator.
Figures 24A and 24B show another buckle variation of the anchor lock element.
Figure 25 shows attachment of a variation of the anchor lock element to the anchor.
Figure 26 shows a variation of the post and anchor lock element having a ratcheting lock.
Figures 27A and 27B show variations of the ratcheting lock.
Figures 28A-28H show actuation of another variation of the ratcheting lock.
Figures 29A-C show a tubular variation of the ratcheting lock element.
Figures 30A-C show a variation of the anchor lock element of Figures 29.
Figures 31A and 31B show a variation of the apparatus of Figures 30 comprising a lock alignment feature.
Figures 32A-F show a method of actuating and adjusting the ratcheting lock of the apparatus of Figures 30.
Figures 33A and 33B show a variation of an anchor/actuator.
Figures 34A-C show detail views of the releasable attachment mechanism of the actuator of Figures 33.
Figures 35A-C show a variation of the releasable attachment mechanism of Figures 34.
Figures 36A-C show another variation of the releasable attachment mechanism.
Figures 37A-C show yet another variation of the releasable attachment mechanism.
Figures 38A-N show variations of a release actuator used in conjunction with the releasable attachment mechanism of Figures 34.
Figures 39A and 39B show detail views of an embodiment of the delivery system/deployment tool.
Figures 40A and 40B show the delivery system/deployment tool of Figures 39 releaseably attached to apparatus 10, and detached from the apparatus.
Figures 41A and 41B show a variation of the delivery system deployment tool of

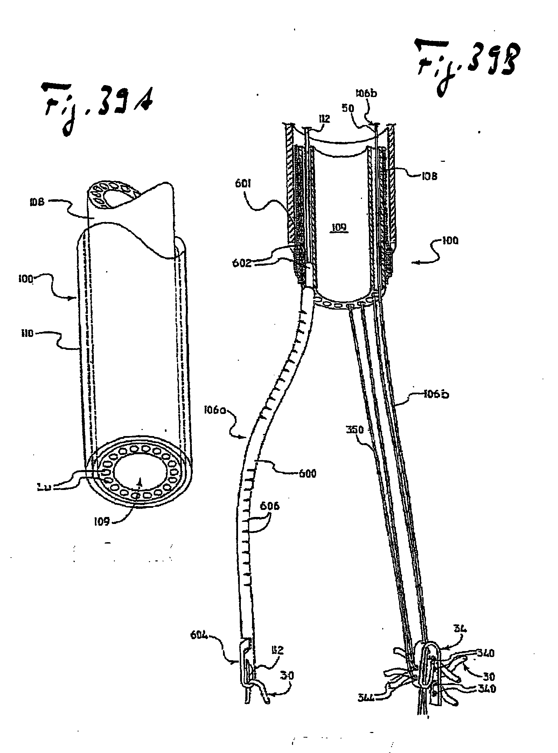

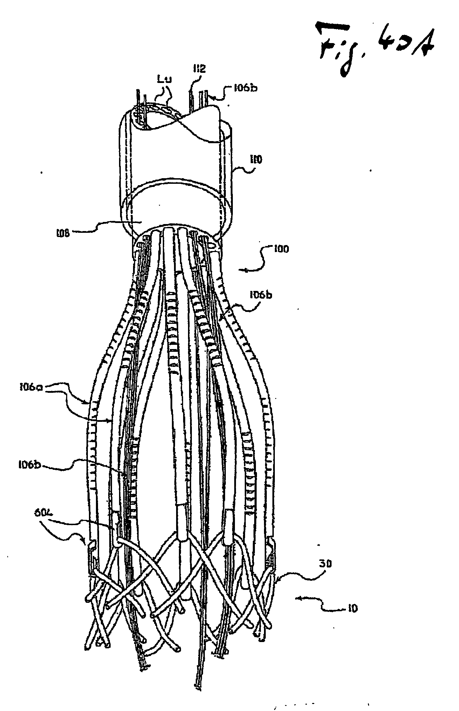

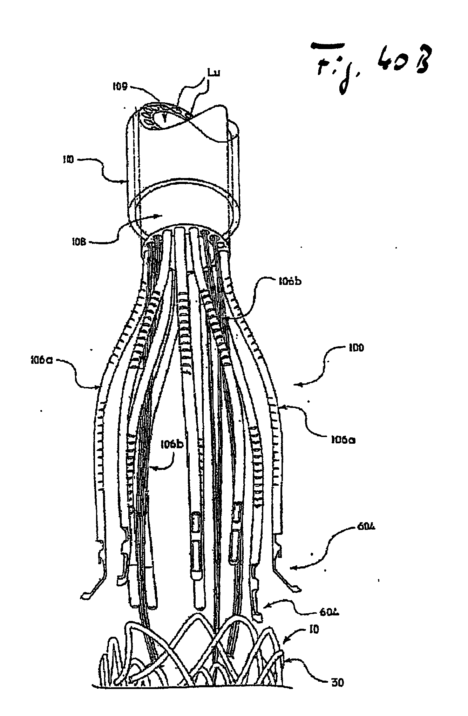

Figures 39 and 40 wherein the actuators extend from a unitary structure.

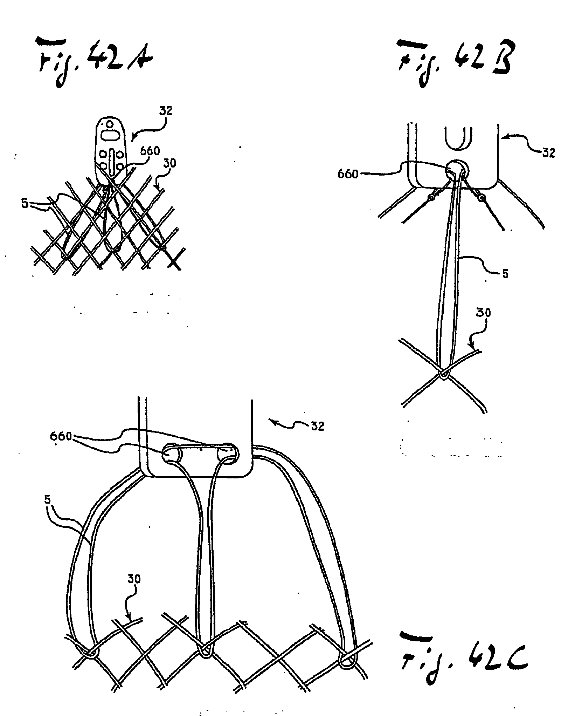

Figures 42A-C show various ways to connect elements to the anchor of the replacement valve apparatus.

### DETAILED DESCRIPTION

The present invention relates to apparatus for endovascularly or percutaneously delivering and deploying a prosthesis, e.g., an aortic prosthesis, within and/or across a patient's native heart valve, referred to hereinafter as replacing the patient's heart valve. A delivery system and/or deployment tool is provided including a sheath assembly and a guidewire for placing the prosthetic apparatus endovascularly within the patient and a user control allowing manipulation of the prosthetic apparatus from external to the patient through the application of a non-hydraulically expanding or non-pneumatically expanding force on the anchor. A hydraulically or pneumatically expanding force would be, for example, a force applied to the anchor by a balloon expanded within the anchor. In certain embodiments, the application of a non-hydraulically expanding or non-pneumatically expanding force could include the use of a hydraulic component transmitting a proximally or distally directed force on an anchor.

The apparatus includes an anchor and a replacement valve. The anchor includes an expandable anchor such as a braid. In preferred embodiments, the expandable braid includes closed edges, but the edges may alternatively be open. The replacement valve is adapted to be secured within the anchor, and as such, be delivered endovascularly to the patient's heart to replace one of the patient's native heart valves. More preferably, the apparatus and methods of the present invention contemplate replacement of the patient's aortic valve.

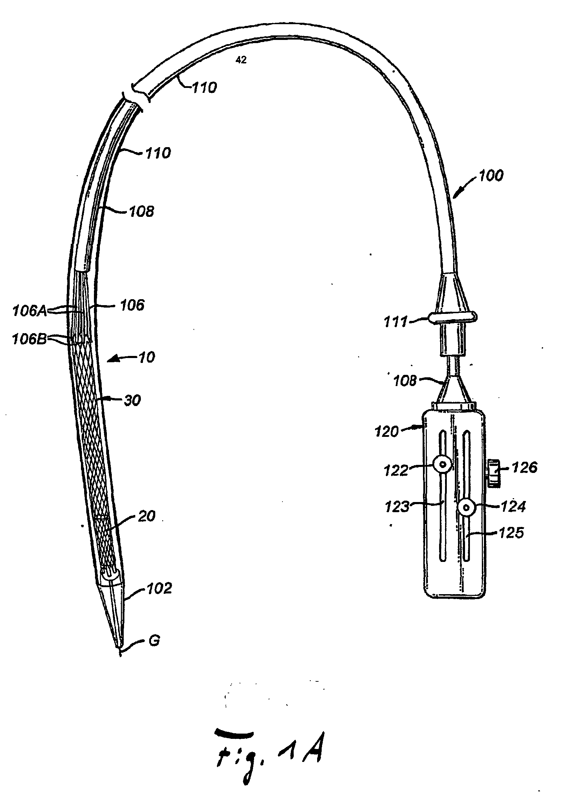

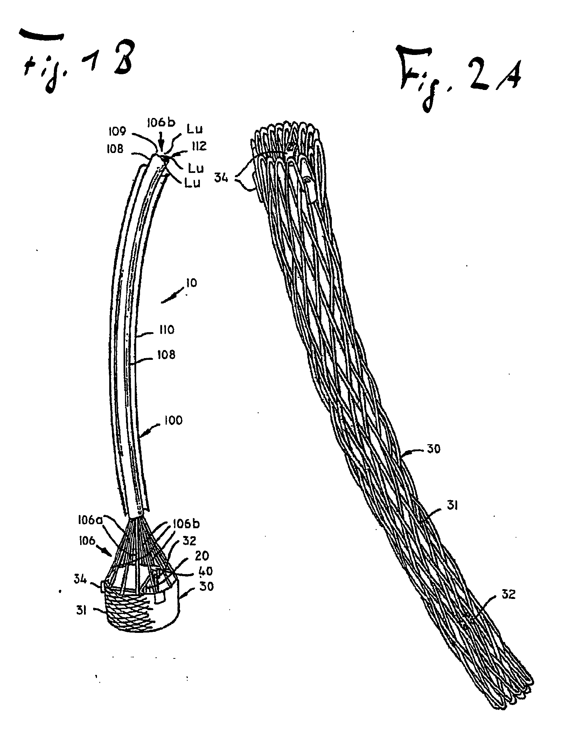

Figures 1A and 1B illustrate one embodiment of a delivery system/deployment tool and apparatus in accordance with the present invention. As seen in Figure 1A, apparatus 10 may be collapsed for delivery within delivery system/deployment tool 100. Delivery system 100 includes guidewire G, nosecone 102, anchor actuation elements 106, multi-lumen shaft or catheter 108 having optional central lumen 109 and a plurality of circumferentially disposed lumens Lu, external sheath 110 having optional proximal handle 111, and confrol handle 120. Nosecone 102 may, for example, be manipulated via a shaft extending through central lumen 109 of multi-lumen catheter 108.

Anchor actuation elements 106 preferably comprise both proximal anchor actuation elements and distal anchor actuation elements. The proximal anchor actuation elements may, for example, comprise actuators 106a that are releasably coupled to a proximal region of anchor 30 of apparatus 10 via releasable attachment mechanisms for manipulating a proximal region of apparatus 10. The distal anchor actuation elements may comprise actuators 106b that are releasably coupled to a distal region of anchor 30 via releasable attachment mechanisms for manipulating the distal region of apparatus 10. In some embodiments, the distal anchor actuation elements may comprise posts or anchor attachment elements 32 of anchor 30 and the releasable attachment mechanisms connecting actuators 106b to posts 32. In an alternative configuration, the proximal anchor actuation elements may be releasably coupled to a proximal region of apparatus 10 through posts and releasable attachment mechanisms for manipulation of a proximal region of the apparatus, while the distal anchor actuation elements may connect to a distal region of anchor 30 via releasable attachment mechanisms to manipulate a distal region of the apparatus. As another alternative, both proximal and distal anchor actuation element may connect to anchor 30 via releasable attachment mechanisms.

In the embodiment shown in Figures 1, actuators 106a may, for example, include stiff finger elements extending from a distal region of multi-lumen shaft 108, while actuators 106b may include control elements (e.g., stands of suture, or metal or polymer wires) which pass through one or more lumens Lu of shaft 108. Release actuators 112 for the releasable attachment mechanisms for both sets of actuators also may pass through one or more lumens Lu of shaft 108. The release actuators may comprise, for example, control elements (e.g., strands of suture, or metal or polymer wires), covers, mandrels, elongated elements, friction surfaces, wrap portions, interference shapes, etc. The release actuators preferably are movable relative to anchor actuation elements 106, e.g., via control handle 120.

Control handle 120 is coupled to multi-lumen shaft 108. Knob 122 disposed in slot 123 may actuate release actuators 112 that couple actuators 106a of anchor actuation elements 106 to apparatus 10. Likewise, knob 124 disposed in slot 125 may actuate release actuators 112 that couple actuators 106b of anchor actuation elements 106 to posts 32 of anchor 30 of apparatus 10. Handle 120 also comprises knob 126 for, e.g., manipulating the actuators 106b to control movement of the distal region of apparatus 10 relative to its proximal region. Conversely, controlled movement of the proximal region of apparatus 10 relative to its distal region may be achieved by holding knob 126 stationary while advancing or retracting handle 120. Knob 126 optionally may move actuators 106b in unison with their concomitant release actuators 112.

Apparatus 10 comprises anchor 30 and replacement valve 20. Anchor 30 preferably comprises a braid. Such braid can have closed ends at either or both its ends. Replacement valve 20 is preferably coupled to the anchor along posts 32, e.g., along a valve attachment structure, such as a tab and/or a plurality of holes. Posts 32, therefore, may function as valve supports and may be adapted to support the replacement valve within the anchor, h the embodiment shown, there are three posts, corresponding to the valve's three commissural attachment points. The posts can be attached to the braid portion of anchor 30. The posts can be attached to the braid's distal end, as shown in Figure 2A, central region, or proximal end. Replacement valve 20 can be composed of a synthetic material and/or may be derived from animal tissue. Replacement valve 20 is preferably configured to be secured within anchor 30.

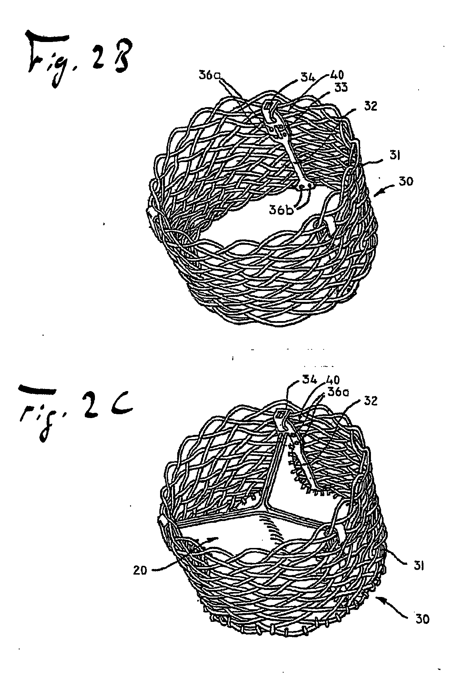

Anchor 30 comprises a plurality of anchor lock elements 34, e.g., buckles 34, attached to its proximal region, one for each post 32. Posts 32 may comprise a lock element that forms a two-part locking mechanism with anchor lock elements 34 for maintaining anchor 30 in a deployed or expanded configuration (e.g., as illustrated in Figures 1B, 2B and 2C).

In this embodiment, anchor 30 is formed from a collapsible and expendable wire braid. Anchor braid 30 is preferably self-expanding and is preferably formed from a material such as Nitinol, cobalt-chromium steel or stainless steel wire using one or more strands of wire. Delivery and deployment of braided anchor 30 is similar to the delivery and deployment of the anchors described in U.S. Patent Appl. Ser. No. 10/746,120. Specifically, in one embodiment described below, during deployment braided anchor 30 is actively foreshortened by proximally retracting the actuators 106b relative to the actuators 106a to expand and lock the anchor in place. In some embodiments, foreshortening may expand anchor 30 to a radially symmetrical, bilaterally symmetrical, or asymmetrical expanded shape. The foreshortening step can include expanding a first region of the anchor to a first diameter and a second region of the anchor to a second diameter larger than the first diameter. A third region may also be expanded to a diameter larger than the first diameter. The expansion of various regions of the anchor (e.g., the distal region) can be especially useful in locating the aortic valve and centering the anchor within it. Preferably, the secured anchor does not interfere with the mitral valve or the ostia. In some embodiments, the anchor is allowed to self-expand prior to the foreshortening step.

As seen in Figures 1, after endovascular delivery through sheath 110 to the vicinity of the patient's native valve (such as the aortic valve), apparatus 10 may be expanded from the collapsed delivery configuration of Figure 1A to the expanded deployed configuration of Figure 1B using delivery system/deployment tool 100. To deploy apparatus 10, external sheath 110 may be retracted relative to apparatus 10 by proximally retracting sheath handle 111 relative to control handle 120. Sheath 110 is thereby removed from the exterior of apparatus 10, permitting the anchor 30 to self-expand. For example, if anchor braid 30 is composed of a shape memory material, it may self-expand to or toward its "at-rest" configuration. This at-rest configuration of the braid can be, for example its expanded configuration, a collapsed configuration, or a partially expanded configuration between the collapsed configuration and the expanded configuration, or some combination. In preferred embodiments, the anchor's at-rest configuration is between the collapsed configuration and the expanded configuration. Depending on the at-rest diameter of the braid and the diameter of the patient's anatomy at the chosen deployment location, the anchor may or may not self-expand to come into contact with the diameter of the patient's anatomy at that location.

In its collapsed configuration, anchor 30 preferably has a collapsed delivery diameter between about 3 to 30 Fr, or more preferably 6 to 28 Fr, or more preferably 12 to 24 Fr. In some embodiments, anchor 30 in its collapsed configuration will have a length ranging from about 5 to about 170 mm, more preferably from about 10 to about 160 mm, more preferably from about 15 to about 150 mm, more preferably from about 20 to about 140 mm, or more preferably from about 25 mm to about 130 mm.

Similarly, in its expanded configuration, anchor 30 preferable has a diameter ranging between about 10 to about 36 mm, or more preferably from about 24 to about 33 mm, or more preferably from about 24 to about 30 mm. In some embodiments, anchor 30 in its expanded configuration will have a length ranging from about 1 to about 50 mm, more preferably from about 2 to about 40 mm, more preferably from about 5 to about 30 mm, or more preferably from about 7 to about 20 mm.

Overall, the ratio of deployed to collapsed/sheathed lengths is preferably between about 0.05 and 0.5, more preferably about 0.1 to 0.35, or more preferably about 0.15 to 0.25. In any of the embodiments herein, anchor 30 in its expanded configuration preferably has a radial crush strength that maintains the anchor substantially un-deformed in response to a pressure of up to about 0.5 atm directed substantially radially inward toward the central axis, or more preferably up to about 2 atm directed substantially radially inward toward the central axis, hi addition, in any of the embodiments herein, the anchor preferably has an axial spring constant of between about 10 to 250 g/cm, more preferably between about 20 to 200 g/cm, or more preferably between about 40 to 160 g/cm. In addition, in any of the embodiments herein, the anchor is preferably adapted to support the replacement valve at the anchor site in response to a differential pressure of up to about 120 mm Hg, more preferably up to about 240 mm Hg, or more preferably up to about 320 mm Hg.

These parameters are not intended to be limiting. Additional parameters within the scope of the present invention will be apparent to those of skill in the art.

As seen in Figure 1B, anchor 30 may be expanded to a fully deployed configuration from a partial deployed configuration (e.g., self-expanded configuration) by actively foreshortening anchor 30 during endovascular deployment. In some embodiments, foreshortening of the apparatus involves applying a distally directed force on the proximal end of the anchor by one or more anchor actuation elements to move the proximal end of the anchor distally while maintaining the position of the distal end of the anchor. For example, the proximal region of anchor 30 may be pushed distally by certain anchor actuation elements 106, e.g., actuators 106a. Alternatively, foreshortening of the apparatus involves applying a proximally directed force on the distal end of the anchor by one or more anchor actuation elements to move the distal end of the anchor proximally while maintaining the position of the proximal end of the anchor. For example, the distal region of anchor 30 may be pulled proximally via a proximally directed force applied by post actuation elements 106b, this force opposed by anchor actuators 106a.

Anchor actuation elements 106 preferably are adapted to expand radially as the anchor expands radially and to contract radially as the anchor contracts radially. Furthermore, proximally or distally directed forces by the anchor actuation elements on one end of the anchor do not diametrically constrain the opposite end of the anchor. In addition, when a proximally or distally directed force is applied on the anchor by the anchor actuation elements, it is preferably applied without passing any portion of a deployment system through a center opening of the replacement valve. This arrangement enables the replacement valve to operate during deployment and before removal of the deployment system.

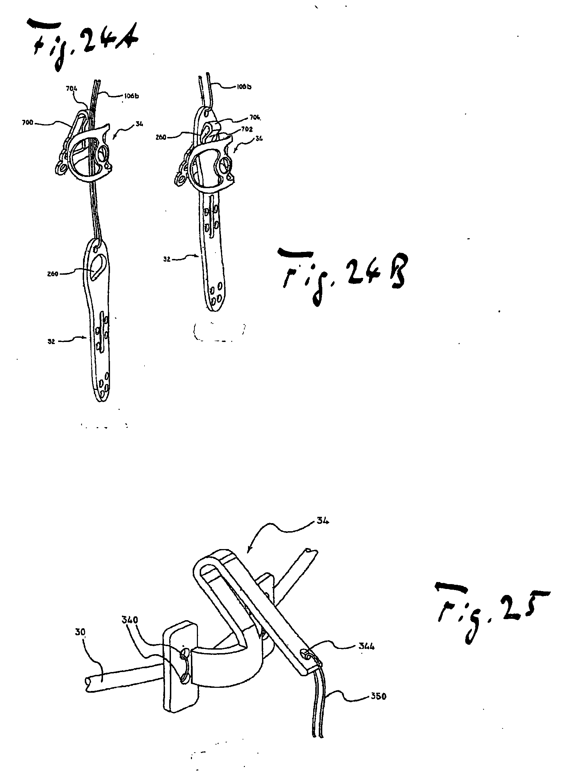

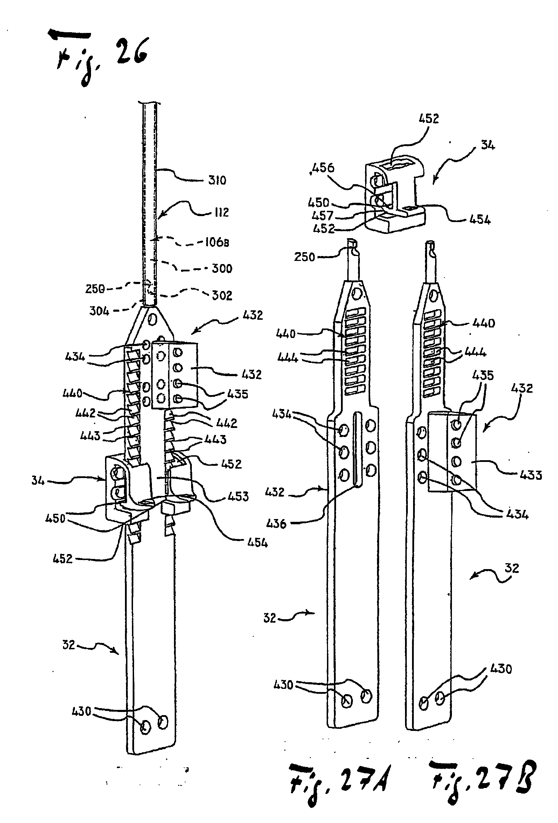

The distal anchor actuation elements may include, for example, actuators 106b and/or release actuators 112 that are controlled, e.g., by control knobs 124 and 126 of confrol handle 120. Similarly, the proximal regions of anchor 30 may be pushed distally via proximal anchor actuation elements, e.g., actuators 106a, at the proximal region of the anchor. The proximal anchor actuation elements facilitate application of a distally directed force to the proximal end of anchor 30 to move or constrain the proximal end of the anchor distally and are controlled through motion of shaft 108 relative to the distal anchor actuation elements. Control knob 122 of control handle 120 may control release actuators 112 for releasing the proximal anchor actuation elements from the braid. The proximal anchor actuation elements may be further adapted to expand as the proximal end of the anchor expands radially during application of a distally directed force on the proximal end of the anchor. Preferably, the proximal anchor actuation elements apply a distally directed force on the proximal end of the anchor system through a plurality of actuators 106a in order to expand the braid of anchor 30. Such braid expansion optionally may be assisted via inflation of a balloon catheter (see Figures 25 and 26) reversibly disposed within apparatus 10, as described in U.S. Patent Appl. Ser. No. 10/746,120.

In the fully deployed configuration, lock elements of posts 32 and anchor lock elements or buckles 34 of anchor 30 may be used to lock and maintain the anchor in the deployed configuration. Apparatus 10 may be repositioned or retrieved from the patient until the lock elements of posts 32 have been interlocked with anchor lock elements 34 of anchor 30 to form lock 40. In one embodiment, actuators 106b and attendant release actuators 112 comprise control elements attached to posts 32 that are threaded through buckles 34 so that the proximally directed force exerted on posts 32 by the control elements during deployment pulls a lock element of posts 32 toward and through buckles 34 to form lock 40. In this manner, the control elements may act as both anchor actuators and lock actuators.

Such lock optionally may be selectively reversible to allow for repositioning and/or retrieval of apparatus 10 during or post-deployment. When the lock is selectively reversible, the apparatus may be repositioned and or retrieved as desired, i.e., even after actuation of lock 40.

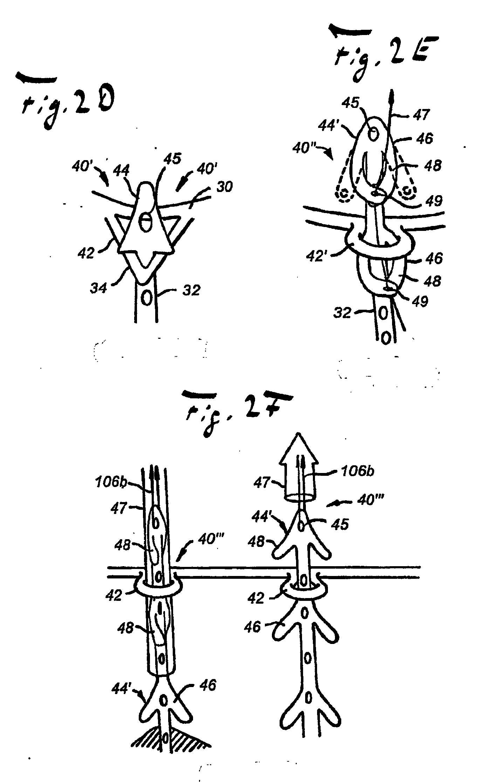

Locks used herein may also include a plurality of levels of locking wherein each level of locking results in a different amount of expansion. For example, the anchor lock elements at the proximal end of the post can have multiple configurations for locking within the buckle wherein each configuration results in a different amount of anchor expansion (see, e.g., Figure 2F). Such locking mechanisms may, for example, comprise ratchets having multiple lock locations. Furthermore, lock alignment features may be provided to facilitate alignment of the post and anchor lock elements, such as a hinge or an oversized width of the post or anchor lock elements. FurtherstiU, lock prevention mechanisms may be provided to preclude locking until desired by a medical practitioner.

When apparatus 10 is placed across a patient's diseased heart valve, anchor 30 may be used to displace the patient's native valve leaflets, and replacement valve 20 will thereafter serve in place of the native valve. After final positioning and expansion, apparatus 10 may be decoupled from delivery system 100 by decoupling the proximal and distal anchor actuation elements 106 from the apparatus via releasable attachment mechanisms, e.g., by decoupling proximal actuators 106a from braided anchor 30 and distal actuators 106b from posts 32 of the anchor via the releasable attachment mechanisms. Moving release actuators 112, e.g., using knobs 122 and 124 of handle 120, may, for example, actuate the releasable attachment mechanisms. Preferably, the releasable attachment mechanisms may be actuated by moving the release actuator(s) less than about 1 inch. After decoupling, delivery system/deployment tool 100 may be removed from the patient, thereby completing endovascular replacement of a patient' s heart valve.

Prior to implantation of replacement valve apparatus described herein, it may be desirable to perform a valvuloplasty on the patient's diseased valve by inserting a balloon into the valve and expanding it using, e.g., saline mixed with a contrast agent, hi addition to preparing the valve site for implant, fluoroscopic viewing of the valvuloplasty will help determine the appropriate size of replacement valve implant to use.

Figures 2A-C show further details of anchor 30 of apparatus 10. Figure 2A shows the apparatus in a collapsed configuration, such as for delivery within a sheath or other lumen or for retrieval and recapture into a sheath or other lumen. Figures 2B and 2C show the anchor and valve in an expanded and locked configuration.

As shown in Figure 2B, anchor 30 illustratively has three posts and three buckles. As seen in Figure 2C, the three leaflets of replacement valve 20 may be, coupled to the three posts 32 along valve support structures. Thus, posts 32 act as valve supports. The posts, unlike the braid, do not collapse or expand. In some embodiments, a post 32 has one or more proximal slots 33, at least one proximal hole 36a and at least one distal hole 36b. Leaflet tissue may, for example, be passed through slot 33 and sutured in place via suture routed through one or more proximal holes 36a. In this manner, slot(s) 33 and hole(s) 36a may form a valve support structure. Alternative valve support structures known in the art for fixing valve leaflets to posts may also be employed.

Posts 32 may be coupled to anchor braid 30 via one or more distal holes 36b. For example, anchor braid 30 may be woven through holes 36b, or a suture or wire may be routed through holes 36b and tied to the braid. Yet another proximal hole (not shown) in post 32 serves as an anchor lock element that interfaces with the anchor lock element provided by buckle 34 to form lock 40. Buckles 34 may likewise be attached to anchor braid 30 via weaving or suturing.

Alternative locks may be used to lock the anchor of the present invention in the foreshortened configuration, as shown, e.g., in Figures 2D-F. Preferably, a lock of the present invention can have multiple locking options such that locking can confer a plurality of amounts of expansion. Furthermore, the locking option can be employed asymmetrically to confer non-cylindrical shapes to the anchor. In Figure 2D, lock 40' comprises male lock element 44 disposed on post 32 and anchor lock element 34 disposed on braided anchor 30. Anchor lock element 34 illustratively comprises triangular protrusion or eyelet 42 of anchor 30. The triangular shape of female lock element 42 may facilitate mating of male lock element 44 with the female lock element without necessitating deformation of the male lock element. One or more holes 45 may be provided through post 32, e.g., for releasably attaching an actuator 106b to the post.

In Figure 2E, lock 40" comprises alternative male lock element 44' having multiple in-line arrowheads 46 along posts 32. Each arrowhead comprises resiliently deformable appendages 48 to facilitate passage through female lock element 42', which illustratively comprises a rounded eyelet. Appendages 48 optionally comprise holes 49, such that releasable lock prevention mechanism 47, illustratively a control wire, may pass through the holes to constrain the appendages in the deformed configuration. To actuate lock 40", one or more arrowheads 46 of male lock element 44' are drawn through female lock element 42', e.g., via a post/lock actuator, and the lock prevention mechanism is removed from holes 49, thereby causing appendages 48 to resiliently expand and actuate lock 40".

Advantageously, providing multiple arrowheads 46 along posts 32 yields a ratchet that facilitates in-vivo determination of a degree of foreshortening and expansion imposed upon anchor 30. Furthermore, optionally constraining appendages 48 of arrowheads 46 via mechanism 47 prevents actuation of lock 40" (and thereby deployment of apparatus 10) even after male element 44' has been advanced through female element 42'. Only after a medical practitioner has removed lock prevention mechanism 47, which constrains appendages 48, is lock 40" fully engaged and is deployment no longer reversible.

Lock 40'" of Figure 2F is similar to lock 40" of Figure 2E, except that holes 49 on appendages 48 have been eliminated, and the lock prevention mechanism comprises overtube or cover 47. Overtube 47 constrains appendages 48 to prevent locking until a medical practitioner has determined that apparatus of the present invention has been foreshortened and positioned adequately at a treatment site. Lock 40'" may, for example, be actuated by applying a proximally-directed force to actuator 106b. Actuator 106b illustratively comprises a control wire releasably disposed through hole 45 in post 32. Lock prevention mechanism 47 then is withdrawn proximally relative to anchor 30, which causes the appendages to resiliently expand, thereby fully actuating lock 40"'.

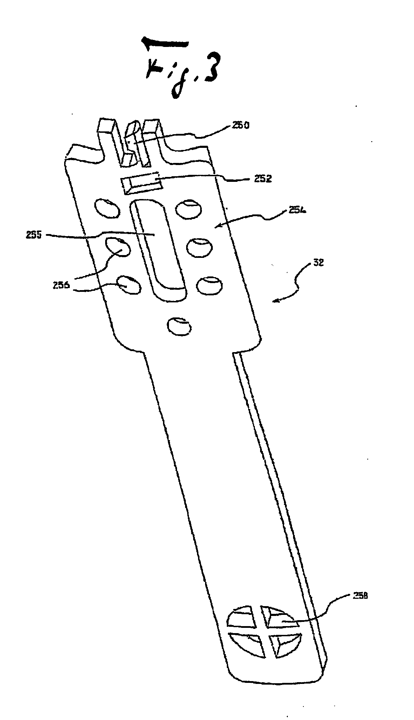

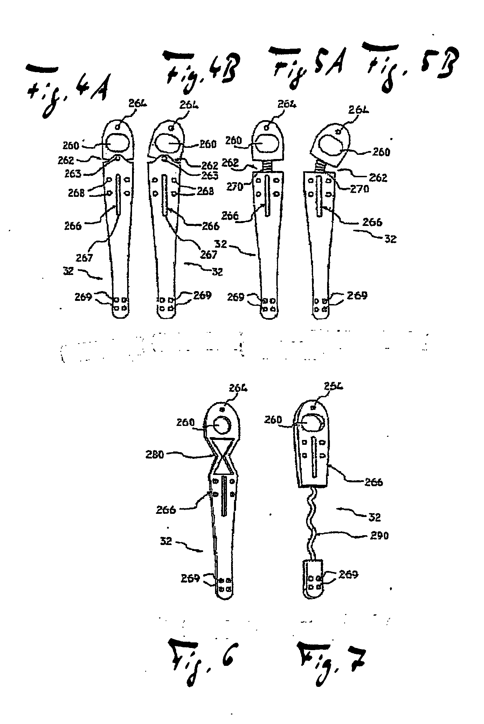

Referring now to Figure 3, a detail view of a variation of post 32 is described, hi Figure 3, post 32 illustratively comprises actuator attachment element 250 for attaching the post to an actuator 106b; post lock element 252, illustratively a slot, for interlocking post 32 with an anchor lock element 34; valve attachment structure 254, comprising slot 255 and a plurality of holes 256, for attaching replacement valve 20 to the post (a tab of the valve may be passed through slot 255, then sewn to the back of the post through holes 256); and braid attachment element 258 for attaching the post to a distal region of anchor 30. The braid of anchor 30 may, for example, be interwoven through braid attachment element 258. Post 32 may be fabricated from a variety of materials, e.g., metallic materials such as stainless steel, and maybe laser cut, die cast, etc. In this variation of post 32, valve 20 is disposed distal of lock element 252. In alternative variations, the valve may be attached to the post proximal of the lock element or in-line with the lock element (i.e., neither proximal nor distal to the lock). Figures 4 provide an alternative variation of post 32. In Figures 4, post 32 comprises lock element 260 having lock alignment feature 262, illustratively hinge 263. Hinge 263 allows lock element 260 to rotate from a position in line with post 32, as in Figure 4A, to a position out of alignment with the post, as in Figure 4B, thereby facilitating alignment with an anchor lock element 34. As shown, post 32 further comprises actuator attachment element 264, illustratively an eyelet, valve support structure 266 having slot 267 and a plurality of holes 268, and braid attachment element 269.

Figures 5 illustrate an alternative variation of lock alignment feature 262 comprising spring 270. As with hinge 263, spring 270 facilitates alignment of post lock element 260 with an anchor lock element 34 by allowing the post lock element to rotate from a position in line with post 32, as in Figure 5A, to a position out of alignment with the post, as in Figure 5B. Spring 270 also applies a restoring force that urges post lock element 260 back into alignment with post 32. Furthermore, spring 270 may facilitate dynamic elongation of post 32 in response to axial tension. This elongation may facilitate axial lengthening of anchor 30 in response to radially inward compression applied to the anchor.

With reference to Figure 6, another variation of post 32 is provided comprising expansion zone 280, which may, for example, comprise a laser cut feature along post 32. Expansion zone 280 facilitates dynamic elongation of post 32 in response to axial tension applied to the post, which facilitates axial lengthening of anchor 30 in response to radially inward compression applied to the anchor. Figure 7 illustrates an alternative expansile element 290 comprising a curved wire or rod that may be elongated and straightened through application of axial tension to facilitate axial lengthening of the anchor in response to radially inward compression applied to the anchor (and thereby axial tension applied to post 32 via interaction between post lock element 260 and an anchor lock element 34).

Element 290 additionally or alternatively may serve as a lock alignment feature. In such a configuration, element 290 optionally may not be expansile. More generally, post 32 may comprise proximal and distal ends connected by a tensile member.

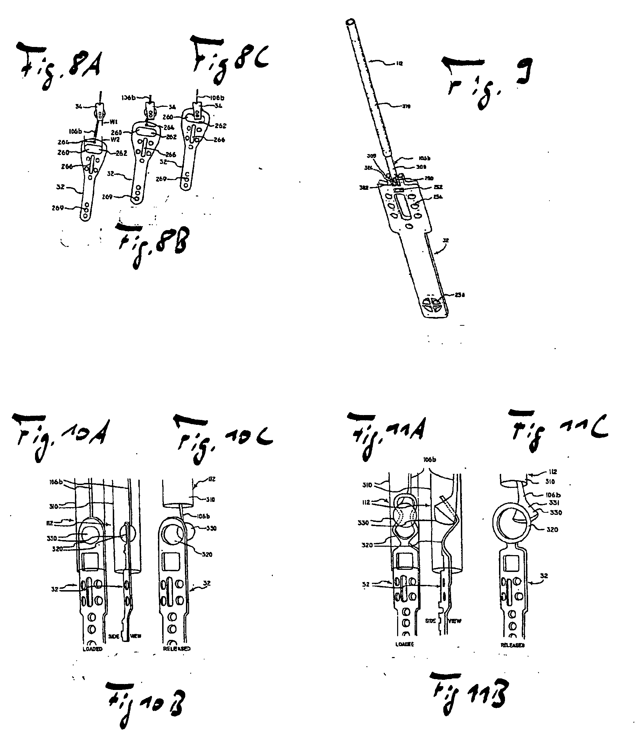

Figures 8 illustrate another variation of post 32 having another alternative lock alignment feature 262. In Figures 8, actuator 106b applies a proximally-directed force which brings post lock element 260 and anchor lock element 34 proximate to one another allowing the system to lock. Anchor lock element 34 defines a lock width Wi. In this embodiment, lock alignment feature 262 comprises post lock element lock area or width W2 that is substantially wider than the lock width Wi, for example, at least about twice as wide. This increased width enhances the probability of interlocking the post and anchor lock elements, even at sharply misaligned angles. In Figures 8, post 32 and anchor lock element 34 are disposed at an illustrative misalignment angle of about 10°.

Referring now to Figure 9, the variation of post 32 of Figure 3 is shown in combination with an illustrative actuator 106b and release actuator 112. In Figure 9, actuator 106b illustratively comprises rod 300 having post attachment element 302 that mates with actuator attachment element 250 of post 32. Angled camming surfaces 304 and 305 of post attachment element 302 and actuator attachment element 250, respectively, form an interface between post attachment element 302 and actuator attachment element 250. Proximal movement of actuator 106b with respect to post 32 is translated by the camming surfaces into a lateral force between the two elements that acts to separate and release post 32 from actuator 106b,. Release actuator 112, illustratively tube 310, may be advanced over actuator 300 to cover the camming surface interface of the post and the actuator 106b, thereby forming a releasable attachment mechanism for securing the post to the actuator even during application of axial tension to the actuator. To separate post 32 from actuator 106b, e.g., after expansion and locking of anchor 30, release actuator 112 may be retracted relative to actuator 106b to the position shown in Figure 9, thereby removing a constraint from camming surfaces 304 and 305 and allowing the post and actuator to be pulled apart. Release actuator 112 preferably is retracted less than about 1 inch relative to the actuator 106b in order to actuate the releasable attachment mechanism, e.g., to remove constraint from camming surfaces 304 and 305.

Referring now to Figures 10, an alternative releasable attachment mechanism for attaching a variation of post 32 to a variation of actuator 106b is described, hi Figures 10A and 10B, post 32 having actuator attachment element 320, illustratively an enlarged proximal opening within the post, is interference fit with post attachment element 330 of actuator 106b, illustratively an enlarged bulb, knob or other distal protrusion of the actuator. The slope of element 330 provides a camming surface that interfaces with an inside surface of opening 320. The angle of the camming interface between element 330 and opening 320 translates proximal movement of actuator 106b with respect to post 32 into a lateral movement between actuator 106b and post 32, thereby separating these elements. Release actuator 112, illustratively tube 310, covers the interference fit releasable attachment mechanism to preclude lateral movement of the post attachment element relative to the actuator attachment element, thereby releasably attaching the post to the actuator 106b. In Figure 10C, tube 310 is retracted relative to the post and actuator, which permits lateral movement between the post and actuator attachment elements, thereby separating actuator 106b from post 32. If tube 310 has not been refracted, of course, proximal movement of actuator 106b moves post 32 and the distal portion of the anchor proximally.

Figures 1 illustrate a variation of the releasable attachment mechanism of Figures 10. In the variation of Figures 11, actuator attachment element 320 of post 32 is deformable from a substantially round profile to an oval or "figure eight" profile by advancement of release actuator 112 over the attachment element. This forms a releasable attachment mechanism. In the deformed profile of Figures 11A and 11B, post attachment element 330 of actuator 106b is interference fit with the deformed actuator attachment element of post 32. In Figure 11C, retraction of release actuator 112 relative to the post and actuator allows actuator attachment element 320 to resiliently resume its un-deformed or at-rest configuration, thereby permitting separation of post 32 from actuator 106b. Actuator attachment element 320 may, for example, be fabricated from a shape memory material, such as Nitinol. A camming surface 331 on post attachment element 330 and a corresponding surface on the inner portion of element 320 translate proximal movement of actuator 106b with respect to post 32 into lateral movement of element 330 with respect to element 320 when release actuator 112 has been retracted.

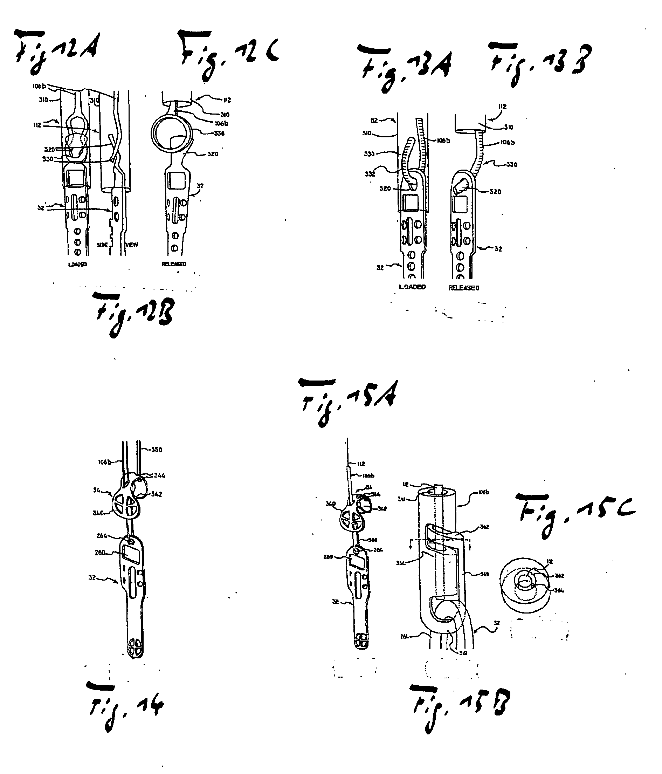

In the variation of Figures 12, post attachment element 330 is deformable (as in Figures 12A and 12B), and anchor attachment element 320 may be interference fit with the post attachment element. Figure 12C shows the post attachment element 330 in its at-rest configuration after tube 310 has been retracted, thereby releasing anchor attachment element 320. As will be apparent, for many or all of the two-part locking or attachment element elements described herein, the position of the elements may be reversed.

In Figures 13, post attachment element 330 comprises wrap portion 332 that may be inserted through anchor attachment element 320, illustratively an eyelet, wrapped backwards, then covered with release actuator tube 310 to constrain the wrap portion 332 in the wrapped configuration, as in Figure 13A. Release actuator tube 310 may be retracted relative to the wrap portion to resiliently or dynamically (e.g., by retracting actuator 106b relative to post 32) reshape the wrap portion to a substantially straight configuration for releasing the attachment between the post and the actuator, as in Figure 13B. Wrap portion 332 preferably is fabricated from a shape memory material, such as Nitinol, or a resilient material, such as spring steel.

Figure 14 shows another variation of the post, actuator and anchor lock element. In Figure 14, post 32 comprises post lock element 260 and actuator attachment element 264, illustratively an eyelet, through which actuator 106b is reversibly disposed. Anchor lock element 34 illustratively comprises a buckle, which may, for example, be formed from a cut tube or a bent resilient material. Anchor lock element 34 comprises anchor or braid attachment element 340 for attaching the buckle to anchor 30, and tab 342 for interlocking the buckle with post lock element 260, which illustratively is a slot formed through post 32. Actuator 106b therefore actuates the post (and therefore the distal end of the anchor to which the post is attached) as well as the anchor lock. Actuator 106b may be released from the post (and therefore from the anchor) by pulling one end of the control wire proximally to draw the control wire through and out of opening 264.

Anchor lock element 34 also comprises optional unlock actuator attachment 344, illustratively a pair of eyelets, through which unlock actuator 350 is releasably coupled to anchor lock element 34. Unlock actuator 350 illustratively comprises a control wire. Upon locking of tab 342 of buckle 34 within slot 260 of post 32, a proximally-directed force applied to unlock actuator 350 may remove the tab from the slot, thereby unlocking buckle 34 and post 32 and permitting the anchor to contract and elongate. Unlocking may be utilized, for example, to reposition or retrieve the anchor and valve apparatus even after the apparatus has been locked in the fully deployed configuration, as described previously with respect to Figures 5.

Figures 15 show another variation of the actuator, the lock actuator and the release actuator. As with other anchor lock elements, anchor lock element 34 in this embodiment is attached to a proximal end of the anchor, and the distal end of post 32 is attached to a distal end of the anchor. The anchor is not shown in Figures 15 for ease of illustration. For the purposes of illustration, the unlock actuator also is not shown in Figures 15.

As shown, actuator 106b actuates both post 32 (and therefore the distal end of the anchor to which the post is attached) and the lock formed between post lock element 260 and anchor lock element 34. In Figure 15A, release actuator 112 passes through actuator 106b to actuate the releasable attachment mechanism between post 32 and actuator 106b. Figure 15B provides a detail view of the releasable attachment mechanism. Actuator 106b comprises wrap portion 360 that passes through actuator attachment element 264 and wraps around the end of post 32. Wrap portion 360 may comprise a shape memory material, such as Nitinol, or a deformable material, e.g., a resiliently deformable material.

Wrap portion 360 further comprises first opening 362 for engaging release actuator 112, illustratively a wire or rod that passes through lumen Lu of actuator 106b. The walls of the lumen act a linear bearing and/or motion guide during advancement and retraction of the release actuator relative to the actuator. Actuator 106b also comprises second opening 364, which may be aligned with first opening 362 to engage release actuator 112, as shown. As seen in the cross-sectional view of Figure 15C, wrap portion 360, and especially the curved portion 361 of the wrap portion, acts as a spring element that urges the first opening out of alignment with the second opening. In this manner, release actuator 112 may be interference or friction fit through first opening 362 and second opening 364. Retraction of the release actuator proximal of the first and second openings may actuate the releasable attachment mechanism to resiliently or dynamically unwrap portion 360 and release actuator 106b from post 32. Wrap and/or curved portion 360/361 of actuator 106b illustratively is disposed at a distal end of the actuator.

As will be apparent to those of skill in the art, the releasable attachment mechanism of Figures 15 may also be utilized to attach a actuator 106a to a braided anchor 30. More generally, wrap portion 360 provides an illustrative first shape on an anchor actuation element 106 that is adapted to mate with a second shape on a post or anchor actuator attachment element (such as element 264 in Figures 15, or a wire of the braid of anchor 30) to substantially prevent relative distal or proximal movement between the anchor actuation element and the anchor. The apparatus further comprises a release actuator adapted to actuate the releasable attachment mechanism. The release actuator is adapted to be moved to permit relative movement between the first shape and the second shape. This relative movement may change the first shape and/or the second shape to a third shape that permits relative distal or proximal movement between the anchor actuation element and the anchor or post. Furthermore, this relative movement may separate the anchor actuation element from the anchor or actuator attachment element.

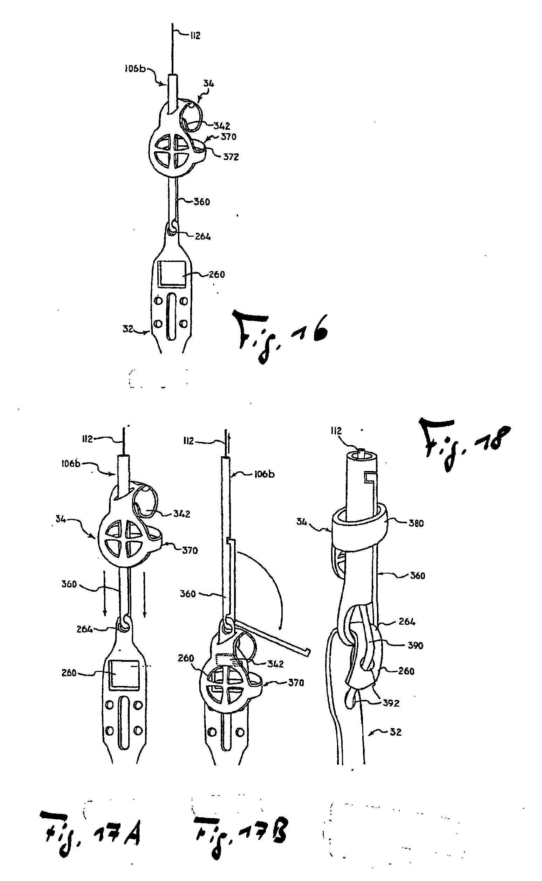

Figure 16 illustrates a variation of the anchor lock element of Figures 15. In Figure 16, anchor lock element 34 comprises lock alignment feature 370. Feature 370 comprises engagement portion 372, illustratively a loop, that is adapted to engage post 32 before engagement of anchor lock element 34 (i.e., before engagement of tab 342 of the anchor lock element) with post lock element 260. Feature 370 ensures alignment of the post and buckle prior to locking. Furthermore, feature 370 adds additional strength to anchor lock element 34 and opposes inwardly-directed forces applied to element 34 when valve 20 of apparatus 10 closes during diastole.

Referring now to Figures 17, actuation of the apparatus of Figure 16 is described. As seen in Figure 17A, anchor lock element 34 is advanced distally relative to post 32, for example, by applying a distally-directed force to the anchor via anchor actuator 106a to move the proximal portion of the anchor distally while maintaining the position of post 32 via actuator 106b. Alternatively or additionally, a proximally-directed force may be applied to post 32 via actuator 106b while maintaining the position of the proximal end of the anchor to move the distal portion of the anchor proximally. Lock alignment feature 370 engages the proximal end of the post prior to interlocking of tab 342 of anchor lock element 34 with post lock element 260, thereby ensuring proper alignment. Continued retraction of post 32 relative to buckle 34 locks the post into the buckle, as shown in Figure 17B. This also expands apparatus 10 to the fully deployed configuration of, e.g., Figures 1B and 2C. Next, release actuator 112 is retracted proximally relative to actuator 106b, which causes wrap portion 360 of the actuator to resiliently or dynamically swing outwards, thereby bringing first opening 362 and second opening 364 out of alignment. Proximal retraction of actuator 106b relative to post 32 removes wrap portion 360 from actuator attachment element 264 of post 32.

Figure 18 shows a variation of the apparatus of Figures 16 and 17. In Figure 18, anchor lock element 34 comprises locking hoop 380, while post lock element 260 comprises a wrapped or curved proximal end of post 32. The curved proximal end also forms actuator attachment element 264. Wrap portion 360 of actuator 106b is wrapped about the curved end of post 32. Release actuator 112, passing through first opening 362 and second opening 364 of actuator 106b, releasably secures this attachment. The release actuator further comprises kink 390 that facilitates passage of the actuator through release actuator attachment elements 392 of post 32, illustratively eyelets. When disposed through elements 392, release actuator 112 further acts as a lock prevention mechanism that precludes locking of the curved proximal end of post 32 with hoop 380 of anchor lock element 34.

In use, the proximal end of post 32 may be retracted through hoop 380 of anchor lock element 34. Release actuator 112 then may be refracted relative to anchor actuator 106b and post 32, such that the release actuator is disposed proximal of attachment elements 392 of the post. Next, post 32 may be allowed to distally advance until its curved proximal end catches and locks against hoop 380 of element 34. Continued retraction of release actuator 112 relative to actuator 106b facilitates separation of the actuator from the post, as described previously.

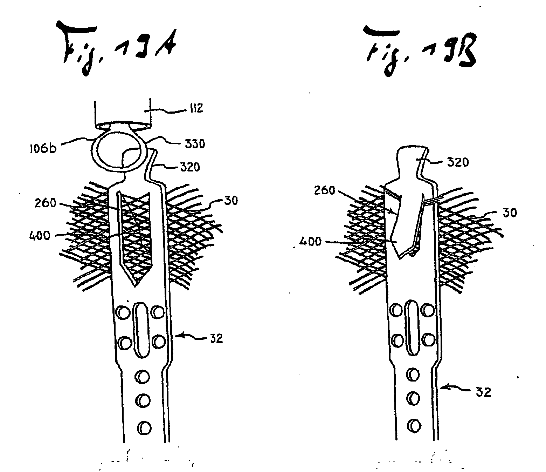

Referring now to Figure 19, an embodiment of post 32 is described that is configured to lock against the braid of anchor 30, as opposed to a separate anchor lock element 34. Post lock element 260 illustratively comprises bent tab 400 that catches against the anchor braid to lock the anchor in a deployed configuration.

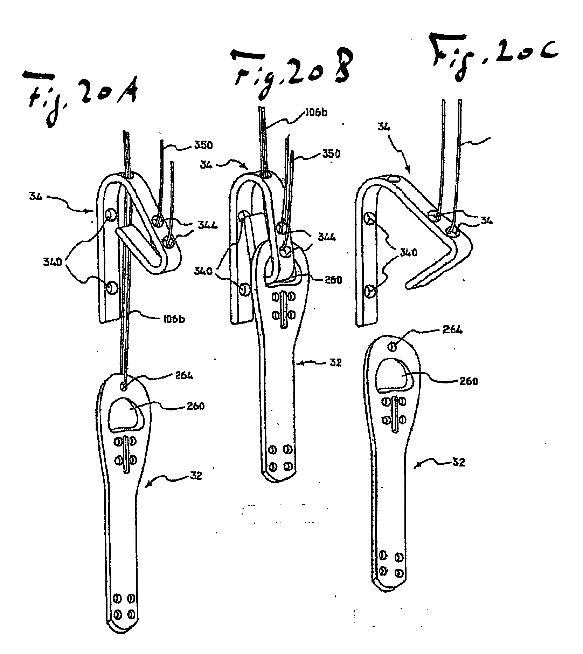

Figures 20 illustrate locking and unlocking of a variation of anchor lock element 34. Anchor lock element 34 of Figures 20 is similar to the buckle variation of element 34 described previously with respect to Figures 14 and 15. However, the variation of Figures 20 is fabricated from a strip of material that is bent to form a wrapped or curved portion. Figure 20A illustrates the apparatus prior to locking, Figure 20B illustrates the locked configuration, and Figure 20C illustrates unlocking through application of a proximally-directed unlocking force to unlock actuator 350.

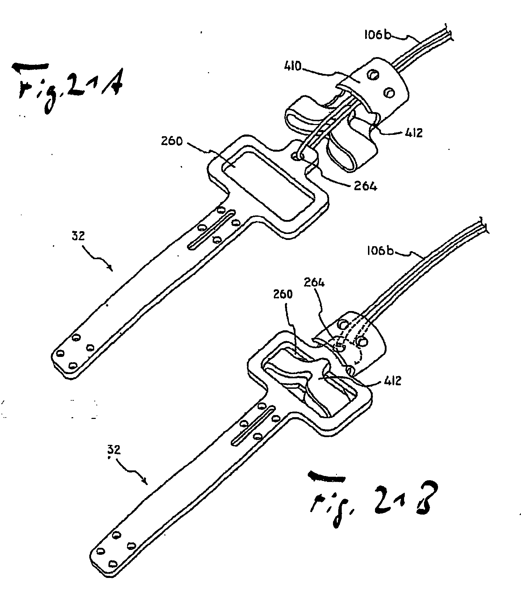

Figures 21 show yet another embodiment of a releasable actuation mechanism. Anchor lock element 34 comprises lock alignment mechanism 410 disposed proximal of locking tab 412. As shown, lock alignment mechanism 410 engages the distal end of post 32 to align the post and the anchor lock element prior to locking of post lock element 260 with tab 412 of anchor lock element 34. Lock alignment mechanism 410 adds additional strength to anchor lock element 34 and opposes inwardly-directed forces applied to element 34 when valve 20 of apparatus 10 closes during diastole. Advantageously, the inwardly-directed forces act to maintain apparatus 10 in the locked configuration. Mechanism 410 optionally may be formed from a cut tube.

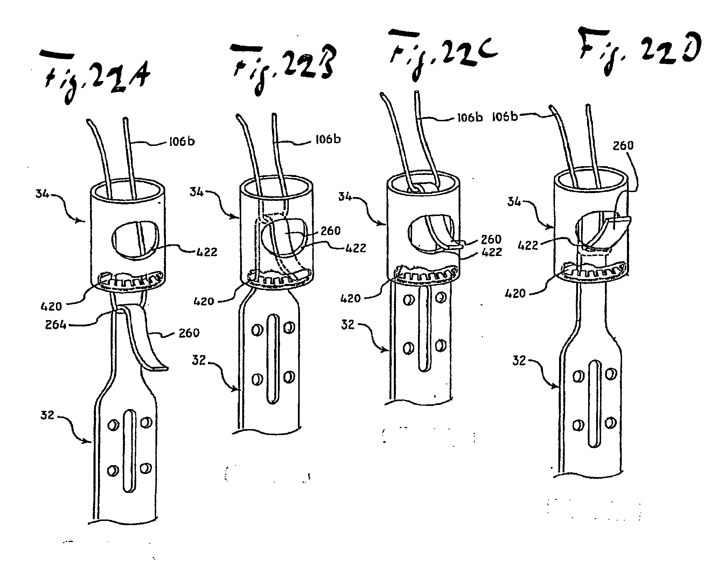

Figures 22 illustrate a variation of anchor lock element 34 that may be formed from a cut tube. As seen in Figures 22A and 22B, element 34 comprises tabs 420 for engaging the curved proximal end of post 32 that forms post locking element 260. In order to lock the post to element 34, the curved distal end of the post is retracted proximally of tabs 420 by the action of proximal tension on post 32 by actuator 106b while element 34 is held stationary, as described above. As it enters anchor lock element 34, the curved end of the post is cammed inward by the engagement of the distal edge of element 34 with the outer surface of the curved end. Once proximal of tabs 420, the curved end of the post moves outward, thereby locking the apparatus and preventing subsequent distal movement of post 32 with respect to element 34. To unlock the apparatus, the curved portion of the post is drawn further proximally by actuator 106b until the tip of the curved portion moves into an opening 422 formed in element 34. As seen in Figures 22C and 22D, resilient distal advancement of the post relative to element 34, e.g., via resilient expansion of the braid of anchor 30, deforms and straightens the curved proximal end of post 32 through a camming engagement of the underside of the curved portion of the post with the inner surface of opening 422, thereby allowing actuator 106b to slide off of post 32, unlocking apparatus 10. The curved portion of post 32 optionally may be formed from a shape memory material, such that the post resumes its curved profile for subsequent relocking after unlocking.

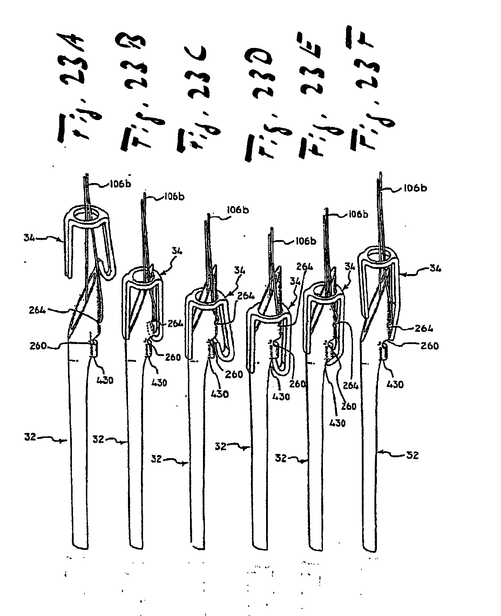

Figures 23 illustrate a variation of post 32 and anchor lock element 32. Anchor lock element 34 illustratively comprises a curved portion 35 that engages and enters the slot of post lock element 260 to lock the anchor as post 32 is drawn proximally into element 34 by actuator 106b. After locking, continued proximal retraction of post 32 by actuator 106b engages the distal end of the curved portion of element 34 with a camming surface 430 of post 32. Resilient distal advancement of post 32 (such as by the resilient contraction and elongation of the anchor to its at-rest configuration) then deforms and straightens the wrapped end of element 34, thereby permitting anchor lock element 34 to separate from post 32, unlocking the apparatus.

Figures 24 and 25 illustrate additional buckle variations of anchor lock element 34. Proximal movement of post 32 into anchor lock element 34 (by, e.g., actuator 106b) engages a bottom surface 702 of a curved portion 700 of element 34 with the proximal end of post 32. Further proximal movement of post 32 with respect to element 34 cams curved portion 700 forward until the curved end 704 of curved portion 700 meets and resiliently moves into opening 260 in post 32, locking the apparatus. The variation of Figures 25 illustrates attachment to the braid of anchor 30 via sutures or the like passed through openings 340 in element 34. The lock is unlockable via unlock actuator 350.

Referring now to Figure 26, an embodiment of a post 32 and anchor lock element 34 with a ratcheting lock is described. Post 32 comprises previously described actuator attachment element 250 that is releasably secured to post attachment element 302 of actuator 106b. (Other releasable attachment mechanisms may alternatively be used.) Post 32 also comprises braid attachment element 430 and valve attachment structure 432. In the variation of Figure 26, valve attachment structure 432 comprises tab 433 that extends from post 32, as well as a plurality of holes 434 through post 32 and a plurality of holes 435 through tab 433. Replacement valve 20 may be attached to post 32 by sewing the valve to the valve attachment structure through holes 434 and/or 435.

Post 32 further comprises ratcheting locking element 440 having a plurality of inclined planes with camming surfaces 442 and friction surfaces 443. The inclined planes are disposed along either side of tab 433 for ratcheting and locking against ratcheting anchor lock element 34. Anchor lock element 34 comprises ratchet teeth 450 on either side of the valve attachment elements that cam against surface 442 and lock against friction surfaces 443 of element 440 of post 32, as post 32 is proximally retracted through element 34. Advantageously, providing multiple rows of inclined plane ratchets along post 32 facilitates interlocking of the post and the element at multiple discrete locations.

Element 34 comprises proximal and distal slots 452 that receive post 32, as well as central longitudinal slot 453 that facilitate passage of tab 433 (and thereby valve 20) therethrough. Actuator 106b may be disposed through slots 452 prior to approximation and locking of the post to anchor lock element 34 in order to facilitate alignment of the post and the anchor lock element. Element 34 may be ratcheted to any position along ratchet lock element 440 to achieve any desired locking configuration and degree of expansion of apparatus 10. Valve attachment structure 432, and thereby replacement valve 20, may be positioned proximal of the ratchet lock post-deployment or in line with the ratchet lock (i.e., neither proximal nor distal to the ratchet lock). Element 34 further comprises unlock actuator attachment(s) 454 for coupling the element to an unlock actuator, e.g., previously described unlock actuator 350, to unlock element 34 by applying a proximally-directed unlocking force that displaces ratchet teeth 450 from friction surfaces 443.

Figures 27 illustrate variations of the apparatus of Figure 26. Ratchet lock elements 440 of posts 32 in Figures 27 comprise a plurality of ratchet slots 444 in which ratchet tooth 450 of anchor lock element 34 may be locked. Ratchet tooth 450 comprises proximal friction surface 456 and distal camming surface 457 to facilitate proximal retraction of a post 32 through slot 452 for ratcheting of camming surface 457 through ratchet slots 444, but to preclude distal advancement of the post once ratchet tooth 450 is engaged within ratchet slots 444 by locking a ratchet slot against friction surface 456. As with the variation of Figure 26, anchor lock element 34 is unlockable and comprises unlock actuator attachment 454. In contrast to the variation of Figure 26, the ratchet lock is disposed proximally of valve attachment structure 432, and thereby proximally of replacement valve 20. In Figure 27A, valve attachment structure 432 comprises slot 436 instead of tab 433.

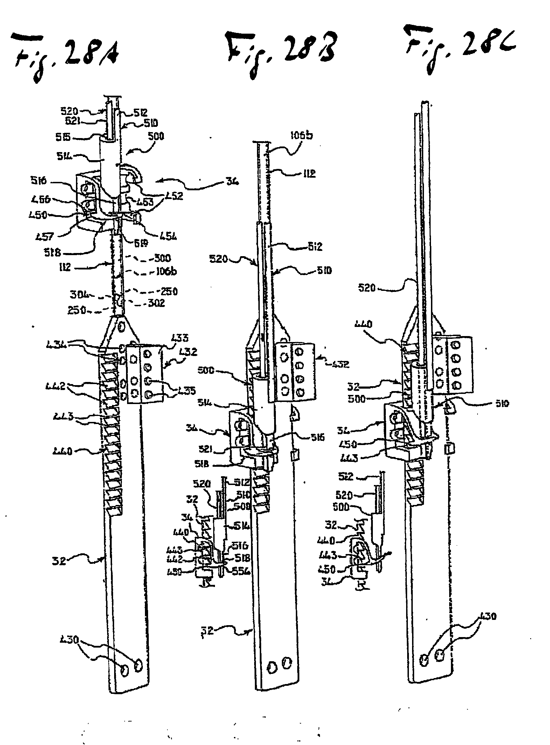

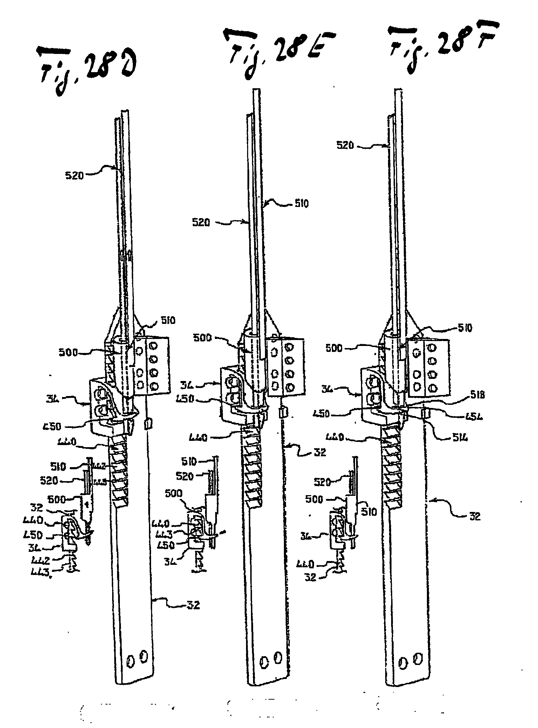

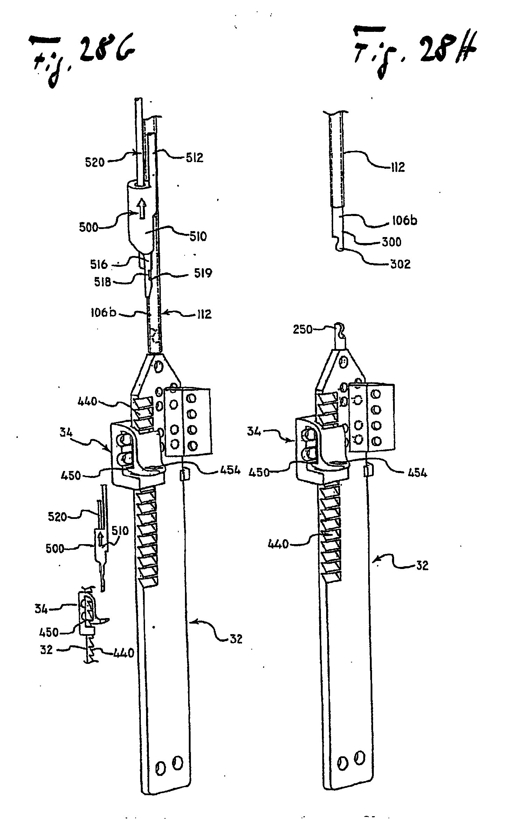

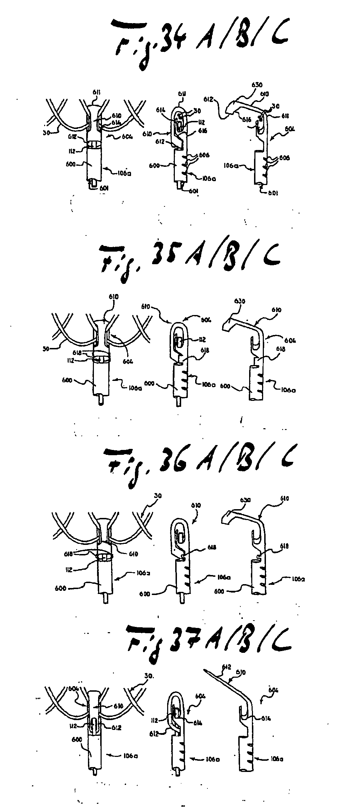

Figures 34 illustrate another variation of the ratchet lock of Figure 26. In Figures 28, ratchet lock elements 440 of post 32 extend along only one edge of the post. Thus, anchor lock element 34 comprises unitary ratchet tooth 450 for camming against surfaces 442 and locking against friction surfaces 443 of elements 440 of post 32, as post 32 is proximally refracted through element 34.

The apparatus of Figures 28 also comprises unlock or adjustment actuator 500 that is releasably attached to anchor lock element 34 along unlock actuator attachment 454. Actuator 500 comprises two independently or concurrently actuable elements: adjustment element 510 and release element 520. Adjustment element 510 comprises elongated member 512 having protrusion 514 with lumen 515, as well as distal extension 516 with notch 518 having optional camming surface 519. Release element 520 comprises elongated member 521, which may, for example, comprise a mandrel, that is configured for passage through lumen 515 of protrusion 514 of adjustment element 510. Elongated members 512 and 521 of actuator 500 preferably extend through delivery system 100 to the exterior of the patient for independent or concurrent advancement and/or retraction by a medical practitioner.

As seen in Figure 28A, notch 518 of adjustment element 510 of actuator 500 maybe positioned within unlock actuator attachment 454 of anchor lock element 34 during deployment of apparatus 10. As seen in Figure 28B, anchor lock element 34 is locked within ratcheting lock elements 440 of post 32 by proximally retracting actuator 106b relative to anchor lock element 34. Release element 520 then may be advanced relative to adjustment element 510 to position elongated member 521 within unlock actuator attachment 454 adjacent distal extension 516 of adjustment element 510. This serves to friction lock or interference fit actuator 500 within attachment 454 along notch 518 of adjustment element 510. Thus, concurrent advancement and/or retraction of the adjustment and release elements of actuator 500 by a medical practitioner causes anchor lock element 34 to move in unison with actuator 500. As will be apparent, actuator 500 alternatively may be friction locked with anchor lock element 34 prior to full deployment of apparatus 10. Furthermore, actuator(s) 500 may assist, or be used in place of, actuators 106a to deploy apparatus 10.

As seen in Figure 28C, the lock formed between anchor lock element 34 and post 32 may be unlocked or adjusted, as desired, by applying a lateral unlocking force to ratchet tooth 450 via actuator 500 that pulls the ratchet tooth away from a friction surface 443 of ratcheting lock elements 440. Actuator 500 then may be distally advanced or, as seen in Figure 28D, proximally retracted relative to ratcheting lock elements 440 and post 32 to further expand or partially collapse anchor 30, respectively (further expansion alternatively may be achieved by further ratcheting ratchet tooth 450 along camming surface 442 of ratcheting lock elements 440, e.g., by further proximally retracting actuator 106b, which is not shown in Figures 28C-F for the sake of clarity). Anchor actuation elements 106 may assist such controlled expansion or collapse anchor 30.

When (re-)positioned at a desired location and/or when a desired degree of locking has been achieved, the lateral unlocking force may be removed from ratchet tooth 450 to again lock anchor lock element 34 to post 32 along ratcheting lock elements 440, as in Figure 28E. To complete deployment of apparatus 10, adjustment actuator 500 and actuator 106b, as well as actuator 106a (not shown), may be separated from the apparatus. In Figure 28F, release element 520 of actuator 500 is proximally refracted relative to adjustment element 510, thereby removing elongated member 521 of release element 520 from unlock actuator attachment 454 of anchor lock element 34. This removes the interference fit between notch 518 and attachment 454. Proximal retraction of actuator 500 relative to anchor lock element 34 detaches adjustment element 510 of actuator 500 from attachment 454 of anchor lock element 34, as in Figure 28G. Optional camming surface 519 along notch 518 may facilitate such detachment. In Figure 28H, actuator 106b is detached from post 32 by retracting release actuator 112 relative to the actuator, as described previously.

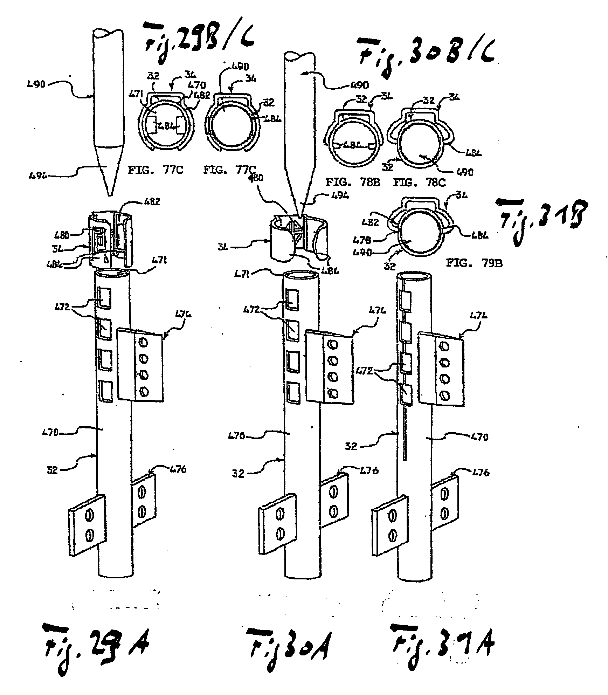

Referring now to Figures 29, another variation of an adjustable ratcheting lock element is described. As seen in Figure 29A, post 32 comprises tube 470 having lumen 471 and ratcheting lock element 472, illustratively a plurality of slots that communicate with lumen 471. Post 32 also comprises valve support structure or attachment element 474 and braid attachment element 476.

Anchor lock element 34, which may be fabricated from a cut tube, comprises a substantially cylindrical structure having braid attachment element 480, lumen 482 and tabs 484. As seen in the top view of Figure 29B, tabs 484 of anchor lock element 34 are configured for locking within the slots of ratcheting lock element 472 of post 32. As seen in the top view of Figure 29C, adjustment actuator 490, illustratively mandrel M having tapered distal end 494 that acts as a camming surface, may be advanced through lumen 481 of anchor lock element 34 and lumen 471 of tube 470 of post 32, to displace tabs 484 from the locking slots of post 32, thereby unlocking the post from the anchor lock element. This facilitates, for example, readjustment of a degree of locking/expansion of apparatus 10, repositioning of apparatus 10, retrieval of apparatus 10, etc.

Figures 30 illustrate a variation of anchor lock element 34 wherein tabs 484 are positioned along a different axis. This may provide a more secure lock between post 32 and anchor lock element 34. Figures 31 illustrate a variation of post 32 configured for use with the variation of anchor lock element 34. In Figures 21, post 32 comprises groove 478 that connects the slots of ratcheting lock element 472. Groove 478 does not communicate with lumen 471 of tube 470 of post 32. Rather, the groove may act as a lock alignment mechanism that guides tabs 484 of anchor lock element 34 along post 32 and ratcheting lock element 472, as seen in the top view of Figure 31B.

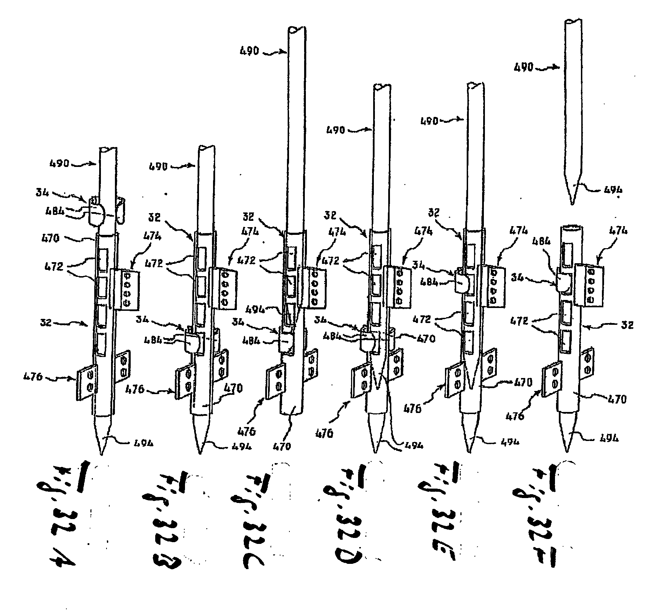

Referring now to Figures 32, a method of actuating the variation of Figures 30 is described. As seen in Figure 32A, adjustment actuator 490 is initially disposed through lumen 482 of anchor lock element 34 and within lumen 471 of post 32. Post 32 then may be proximally retracted relative to anchor lock element 34, e.g., via actuator 106b (not shown). In Figure 32B, actuator 490 serves as a lock prevention mechanism that precludes locking of tabs 484 within ratcheting lock element 472. In Figure 32C, actuator 490 is retracted relative to post 32 and anchor lock element 34, which opens up lumen 471 of tube 470 and allows tabs 484 to pass through the slots of ratcheting lock element 472, thereby locking the post to the anchor lock element. In Figure 32D, actuator 490 is re-advanced within lumen 471, such that tapered distal end 494 of mandrel M serves as a camming surface that urges tabs 484 out of lumen 471 as the actuator is advanced. This unlocks the post from the anchor lock element to facilitate adjustment, repositioning or retrieval of apparatus 10. In Figure 32E, a degree of locking/expansion of the apparatus is adjusted by repositioning anchor lock element 34 relative to post 32, and thereby tabs 484 relative to ratcheting lock element 472. When properly adjusted, actuator 490 may be removed from lumen 471 of tube 470 of post 32, as in Figure 32F. Tabs 484 resiliently return to the locked configuration within the slots of ratcheting lock element 472.

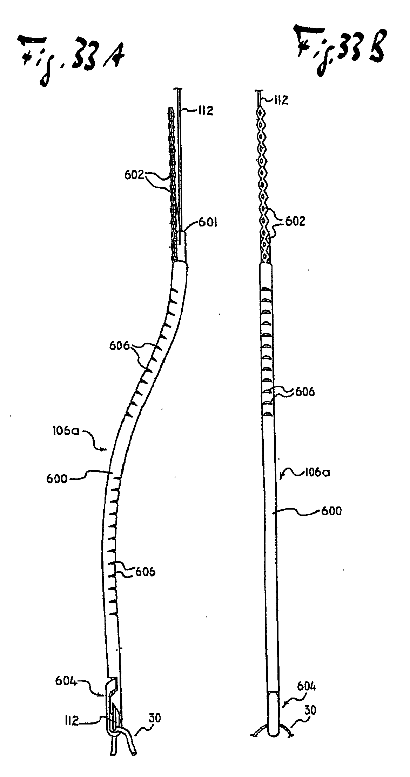

Referring now to Figures 33, an embodiment of anchor actuator 106a is described. Actuator 106a comprises elongated member 600 having proximal extension 602 that may be attached, for example, to previously described multi-lumen shaft or catheter 108 of delivery system/deployment tool 100 (see Figures 49), e.g., via epoxy, UV curing, etc. Lumen 601 extends through elongated member 600 from proximal extension 602 to releasable attachment mechanism 604. Releasable attachment mechanism 604 releasably attached actuator 106a to the braid of anchor 30. The mechanism comprises release actuator 112 and illustratively is similar to the previously described releasable attachment mechanism of Figures 15-17. Release actuator 112, illustratively a mandrel, passes through a lumen Lu of multi-lumen shaft 108 and then through lumen 601 of actuator 106a to mechanism 604.

Actuator 106a further comprises shaping features 606 that affect a shape of the anchor actuator when an anchor actuation force is applied to anchor 30. These features may comprise, for example, reduced diameter portions of the actuator, reduced wall thickness portions of the actuator and/or slits formed in the anchor actuator. Application of an anchor actuation force may, for example, provide actuator 106a with the profile seen in Figure 33A. This profile may facilitate expansion of anchor 30/apparatus 10. As will be apparent, shaping features may be provided with any anchor actuation elements 106, including any of the previously described variations of actuators 106b.

As seen in Figures 34, releasable attachment mechanism 604 comprises wrap portion 610 that may, for example, pass through the braid of anchor 30 and wrap around the proximal end of the anchor. Wrap portion 610 may comprise a shape memory material, such as Nitinol, or a deformable material, e.g., a resiliently deformable material. The wrap portion comprises first opening 612 for engaging release actuator 112. The walls of lumen 601 of elongated member 600 may act as a linear bearing and/or motion guide during advancement and refraction of the release actuator relative to the actuator. Actuator 106a also comprises second opening 614, which may be aligned with first opening 612 to engage release actuator 112, as shown. Wrap portion 610, and especially curved portion 611 of the wrap portion, acts as a spring element that urges the first opening out of alignment with the second opening to engage and hold release actuator 112 in place.

As seen in Figure 34C, when the release actuator is retracted proximally relative to the actuator, wrap portion 610 resiliently or dynamically swings outwards. Thereafter, proximal retraction of anchor actuator 106a relative to anchor 30 detaches wrap portion 610, and thereby actuator 106a, from the anchor. Surface 616 of wrap portion 610 may act as a camming surface as the inner surface of wrap portion 610 slides along the anchor braid 30 to facilitate such detachment.

In this manner, release actuator 112 may be interference or friction fit through first opening 612 and second opening 614. Retraction of the release actuator proximal of the first and second openings actuates releasable attachment mechanism 604 to resiliently or dynamically unwrap portion 610 and release actuator 106a from anchor 30. Wrap portion 610 of actuator 106a illustratively is disposed at a distal end of the actuator.

With reference to Figures 35, a variation of releasable attachment mechanism 604 is described. In Figures 35, wrap portion 610 illustratively comprises tabs 618 that act as an alignment mechanism for aligning the wrap portion of mechanism 604 with elongated member 600. This may facilitate advancement of release actuator 112 through mechanism 604. Figures 36 illustrate a variation of tabs 618 wherein the tabs are rounded. This may reduce friction, provide an afraumatic surface, etc. Additional shapes for tabs 618 will be apparent. Alternatively, tabs 618 may act as spring elements which are loaded when element 630 is seated, as shown in Figure 36B. In this configuration tabs 618 apply a force directed towards element 630 such that 630 will be ejected when element 112 is refracted. In this way tabs 618 apply a restraining force on element 112 which reduces the risk of an early release.

Figures 37 illustrate a variation of wrap portion 610 that comprises a substantially straight distal region in an at-rest configuration, as seen in Figure 37C. It is expected that providing a substantially straight distal region along wrap portion 610 may facilitate detachment of actuator 106a from anchor 30, i.e., may reduce a risk of snagging the wrap portion along the braid of the anchor. The wrap portion may be resiliently deformed for passage of release actuator 112 through first opening 612, as in Figures 37A and 37B.

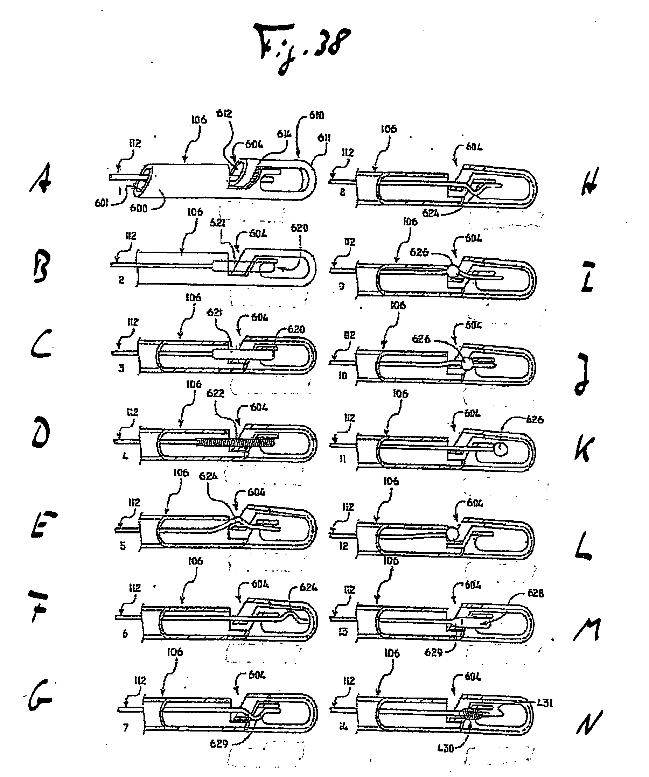

Referring now to Figures 38, variations of release actuator 112 for use with releasable attachment mechanism 604 are described. In Figure 38A, the release actuator comprises a simple mandrel. In Figures 38B and 38C, the release actuator comprises protrusion 60 having friction surface 621. In Figure 38D, actuator 112 comprises coil 622. In Figures 38E-H, the actuator comprises kink 624, which may act as a camming surface, as shown. The kink may also provide tactile feedback to a medical practitioner. In Figures 38I and 38J, the release actuator comprises ball or knob 626 disposed proximal of the actuator's distal end. In Figures 38K and 38L, ball 626 is disposed at the distal end of actuator 112. The ball may act as a camming surface. In Figure 38M, actuator 112 comprises protrusion 628 having proximal camming surface 629. In Figure 38N, the actuator comprises oblong protrusion 430 having friction surface 431. Additional variations of actuator 112 will be apparent.

Referring now to Figures 39, an embodiment of delivery system/deployment tool 100 is described. Figure 39A provides a detail view of multi-lumen catheter 108 and sheath 110. As discussed previously catheter 108 comprises central lumen 109 and a plurality of circumferentially-disposed lumens Lu.

As seen in Figure 39B, actuator 106a is coupled to catheter 108 via proximal extension 602, such that lumen 601 is coaxially disposed within a lumen Lu of the catheter. Release actuator 112 extends through lumens Lu and 601. Actuator 106a is distally attached to the braid of anchor 30 along releasable attachment mechanism 604. For the sake of clarity, a single actuator 106a is shown in Figure 39B, but multiple such actuators preferably are provided, as in Figures 40 described hereinafter.

Figure 39B also illustrates actuator 106b. The actuator extends through a lumen Lu of catheter 108 and through anchor lock element 34 to post 32 (not shown). Unlock actuator 350 is also provided and extends through a lumen Lu to unlock actuator attachment 344 of anchor lock element 34. Anchor lock element 34 illustratively comprises the variation described previously with respect to Figures 20. The element is attached to the braid of anchor 30 along anchor, attachment elements 340. As with actuator 106a, a single anchor lock element 34 and actuator 106b are shown in Figure 39B. This is only for the sake of clarity, and multiple such actuators may be provided, e.g., three actuators.

Referring now to Figures 40, delivery system/deployment tool 100 is shown with a plurality of actuators 106a and actuators 106b for releasable attachment to anchor 30 of apparatus 10. In Figure 40A, anchor actuation elements 106a are coupled to the anchor. In Figure 40B, the elements are decoupled from the anchor.

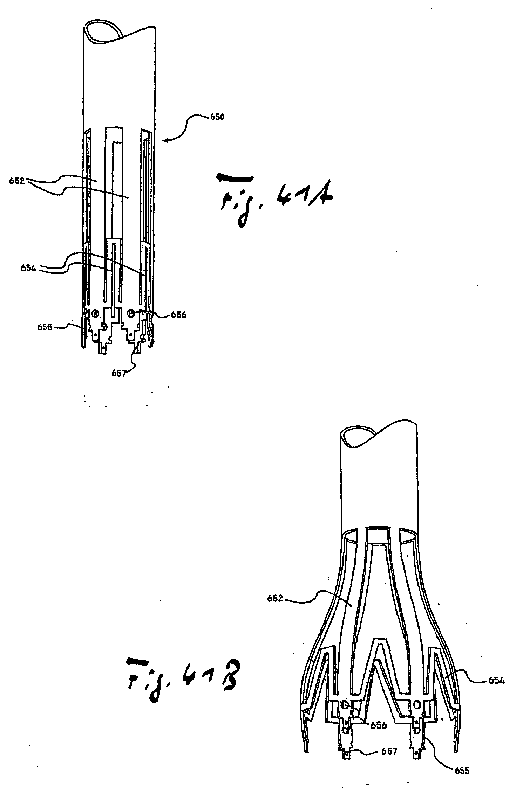

With reference now to Figures 41, a variation of the delivery system/deployment tool of Figures 39 and 40 is described comprising a plurality of arms or actuators that extend from a unitary structure. Unitary structure 650, which may extend from a distal region of multi-lumen shaft 108, is preferably fabricated from a laser-cut tube. Structure 650 comprises a plurality of circumferentially disposed arms 652 that serve as actuators. Expansile elements 654 may be disposed between arms 652 and facilitate constraint of the arms radially outward or inward with respect to other arms as the anchor reshapes. Figure 41A shows the arms in a radially collapsed configuration, and Figures 41B shows the arms in a radially expanded configuration. Wrap portions 655 are adapted to wrap around the proximal portion of an anchor braid. Openings 656 and 657 are formed in wrap portions 655 to engage a release actuator, as described in embodiments above.

Referring now to Figures 42, various ways to connect elements to the braid of anchor 30 of replacement valve apparatus 10 are described. In Figure 42A, a post 32 having a single braid attachment hole 660 is attached to anchor 30 along three separate intersections of the braid via suture S. Figure 42B provides a detail view of one exemplary technique for routing the suture between hole 660 and anchor 30. Figure 42C illustrates a variation of the attachment, wherein post 32 comprises multiple braid attachment holes 660. As will be apparent, elements other than posts 32 may be attached to anchor 30 in the manner described, for example, anchor lock elements 34 may be attached in a similar manner.

As described in more detail in U.S. Patent Appl. Ser. No. 10/746,280, the distal region of anchor 30 may be pulled proximally via a proximally directed force applied to posts 32 via a distal deployment system interface. The distal deployment system interface is adapted to expand radially during application of a proximally directed force on the distal end of the anchor.

The distal deployment system interface may include confrol actuators that are controlled, e.g., by control knob 122 of confrol handle 120. Similarly, the proximal regions of anchor 30 may be pushed distally via a proximal deployment system interface at the proximal end of the anchor. The proximal deployment system interface is adapted to permit deployment system to apply a distally directed force to the proximal end of anchor 30 through, e.g., fingers 106, which are controlled by, e.g., Control knob 124 of confrol handle 120. The proximal deployment system interface may be further adapted to expand radially during application of a distally directed force on the proximal end of the anchor. Preferably, the proximal deployment system interface is adapted to permit deployment system to apply a distally directed force on the proximal end of the anchor system through a plurality of deployment system fingers or actuators 160. Such expansion optionally may be assisted via inflation of a balloon catheter (not shown) reversibly disposed within apparatus 10, as described in U.S. Patent Appl. Ser. No. 10/746,280.

Once anchor 30 is fully deployed, posts 32 and buckles 34 of anchor 30 may be used to lock and maintain the anchor in the deployed configuration. In one embodiment, the control actuators attached to posts 32 are threaded through buckles 34 so that the proximally directed force exerted on posts 32 by the control actuators during deployment pulls the proximal locking end of posts 32 toward and through buckles 34. Such lock optionally may be selectively reversible to allow for repositioning and/or retrieval of apparatus 10 during or post-deployment. Apparatus 10 may be repositioned or retrieved from the patient until the two-part locking mechanism of posts 32 and buckles 34 of anchor 30 have been actuated. When the lock is selectively reversible, the apparatus may be repositioned and/or retrieved as desired, e.g., even after actuation of the two-part locking mechanism. Once again, further details of this and other anchor locking structures may be found in U.S. Patent Appl. Ser. No. 10/746,280. Locking mechanisms used herein may also include a plurality of levels of locking wherein each level of locking results in a different amount of expansion. For example, the proximal end of the post can have multiple configurations for locking within the buckle wherein each configuration results in a different amount of anchor expansion.

Prior to implantation of replacement valve apparatus described herein, it may be desirable to perform a valvuloplasty on the patient's diseased valve by inserting a balloon into the valve and expanding it using, e.g., saline mixed with a contrast agent. In addition to preparing the valve site for implant, fluoroscopic viewing of the valvuloplasty will help determine the appropriate size of replacement valve implant to use.

## Claims

1. Apparatus for endovascularly replacing a patient's heart valve comprising:
a sheath (110);
a deployment tool (100) comprising a plurality of anchor actuation elements (106);
a replacement valve (20) configured to be disposed within the sheath (110) for delivery to a vicinity of the heart valve; and
an expandable anchor (30) configured to be disposed within the sheath (110) for delivery to the vicinity of the heart valve, to be deployed from the sheath, to be expanded by the deployment tool to contact tissue at an anchor site and to be retrieved back into the sheath after having been expanded,
**characterized in that** the anchor actuation elements comprise a plurality of distal anchor actuation elements (106b) adapted to apply an actuation force on a distal portion of the anchor (30), and
**in that** the distal anchor actuation elements each comprises a first member (32) attached to the distal portion of the anchor and a second member (34) releasably attachable to the first member.

2. The apparatus of claim 1, wherein the first members each comprise a lock element adapted to lock the anchor in a deployed state, and further comprising a lock actuator adapted to apply a locking force to lock the anchor in the deployed state.

3. The apparatus of claim 1 or 2, further comprising an unlocking actuator adapted to unlock the anchor.

4. The apparatus of one of the preceding claims, wherein the anchor actuation elements comprise a plurality of proximal anchor actuation elements adapted to apply an actuation force on a proximal portion of the anchor.

5. The apparatus of one of the preceding claims, wherein the first members each comprise a post adapted to support the valve within the anchor.

6. The apparatus of claim 5, wherein at least one post comprises valve attachment structure, the replacement valve being attached to the post via the valve attachment structure.

7. The apparatus of one of the preceding claims, wherein the lock element is further adapted to engage an anchor element to lock the anchor in the deployed shape.

8. The apparatus of one of the preceding claims, wherein the apparatus further comprises anchor lock elements attached to the anchor and adapted to mate with the lock element of each first member to lock the anchor in the deployed shape.

9. The apparatus of claim 8, wherein the anchor lock elements each comprise a camming surface adapted to engage the first member and to move at least a portion of the anchor lock element in response to a lock actuation force.

10. The apparatus of claim 8 wherein at least one first member lock element and at least one anchor lock element are adapted to mate at a plurality of mating configurations.

11. The apparatus of claim 10, wherein the at least one first member lock element comprises a plurality of ratchets adapted to engage a tooth portion of the at least one anchor lock element.

12. The apparatus of one of the preceding claims, wherein the anchor lock elements each comprise a buckle.

13. The apparatus of one of the preceding claims, wherein the first member lock element further comprises a lock alignment feature adapted to align the first member lock element with the anchor lock element.

14. The apparatus of one of the preceding claims, wherein the distal anchor actuation element comprises the lock actuator.

15. The apparatus of claim 3 wherein the unlocking actuator comprises a proximal pull actuator, or wherein the unlocking actuator comprises a distal push actuator.

16. The apparatus of one of the preceding claims, further comprising a lock prevention mechanism.

## Patentansprüche

1. Vorrichtung zum endovaskulären Ersetzen einer Herzklappe eines Patienten umfassend:
eine Hülle (110);
ein Einsetzwerkzeug (100) umfassend eine Vielzahl von Befestigungs-Betätigungselementen (106);
eine Ersatzklappe (20), ausgelegt um in der Hülle (110) angeordnet zu werden für das Zuführen in die Nähe einer Herzklappe; und
eine expandierbare Befestigung (30), ausgelegt um in der Hülle (110) angeordnet zu werden für das Zuführen in die Nähe einer Herzklappe, um von der Hülle aus eingesetzt zu werden, um mit dem Einsetzwerkzeug expandiert zu werden, um Gewebe an einer Befestigungsstelle zu berühren und um nach einer Expansion wieder in die Hülle zurückgeholt zu werden,
**gekennzeichnet dadurch, dass** die Befestigungs-Betätigungselemente eine Vielzahl von distalen Befestigungs-Betätigungselementen (106b) umfasst, die angepasst sind, um eine Betätigungskraft an einen distalen Teil der Befestigung (30) anzulegen,
und dadurch, dass die distalen Befestigungs-Betätigungselemente je ein erstes Teil (32), angebracht an dem distalen Teil der Befestigung, und ein zweites Teil (34), lösbar angebracht an dem ersten Teil, umfasst.

2. Die Vorrichtung nach Anspruch 1, wobei das erste Teil je ein Sicherungselement umfasst, das angepasst ist, die Befestigung in einem eingesetzten Zustand zu sichern, und weiter umfassend eine Sicherungsbetätigung, angepasst um eine Sicherungskraft anzulegen, um die Befestigung im eingesetzten Zustand zu sichern.

3. Die Vorrichtung nach Anspruch 1 oder 2, weiter umfassend eine Entsicherungsbetätigung, angepasst um die Befestigung zu entsichern.

4. Die Vorrichtung nach einem der vorhergehenden Ansprüche, wobei die Befestigungs-Betätigungselemente eine Vielzahl von proximalen Befestigungs-Betätigungselementen umfasst, die angepasst sind um eine Betätigungskraft an einem proximalen Teil der Befestigung anzulegen.

5. Die Vorrichtung nach einem der vorhergehenden Ansprüche, wobei die ersten Teile je einen Stab umfassen, der angepasst ist, die Klappe in der Befestigung zu unterstützen.

6. Die Vorrichtung nach Anspruch 5, wobei zumindest ein Stab eine Klappenanbaustruktur umfasst, wobei die Ersatzklappe über die Klappenanbaustruktur an den Stab angebracht wird.

7. Die Vorrichtung nach einem der vorhergehenden Ansprüche, wobei das Sicherungselement weiter angepasst ist mit einem Befestigungselement einzugreifen, um die Befestigung in der eingesetzten Form zu sichern.

8. Die Vorrichtung nach einem der vorhergehenden Ansprüche, wobei die Vorrichtung auch Befestigungs-Sicherungselemente umfasst, angebracht an der Befestigung und angepasst um mit dem Sicherungselement von jedem ersten Teil zu koppeln, um die Befestigung in der eingesetzten Form zu sichern.

9. Die Vorrichtung nach Anspruch 8, wobei die Befestigungs-Sicherungselemente je eine Eingriffoberfläche umfassen, die angepasst ist mit dem ersten Teil einzugreifen und um zumindest einen Teil des Befestigungs-Sicherungselements in Reaktion auf eine Sicherungs-Betätigungskraft zu bewegen.

10. Die Vorrichtung nach Anspruch 8, wobei zumindest ein Sicherungselement eines ersten Teils und zumindest ein Befestigungs-Sicherungselement angepasst sind, in einer Vielzahl von Koppelkonfigurationen zu koppeln.

11. Die Vorrichtung nach Anspruch 10, wobei zumindest ein Sicherungselement eines ersten Teils eine Vielzahl von Sperrvorrichtungen umfasst, die angepasst sind um mit einem Verzahnungsteil von dem zumindest einen Befestigungs-Sicherungselement einzugreifen.

12. Die Vorrichtung nach einem der vorhergehenden Ansprüche, wobei jedes Befestigungs-Sicherungsselement eine Schnalle umfasst.

13. Die Vorrichtung nach einem der vorhergehenden Ansprüche, wobei das Sicherungselement des ersten Teils weiter eine Sicherungsausrichtungseinrichtung umfasst, die angepasst ist, das Sicherungselement des ersten Teils mit dem Befestigungs-Sicherungsselement auszurichten.

14. Die Vorrichtung nach einem der vorhergehenden Ansprüche, wobei das distale Befestigungs-Betätigungselement die Sicherungsbetätigung umfasst.

15. Die Vorrichtung nach Anspruch 3, wobei die Entsicherungsbetätigung eine proximale Zugbetätigung, oder wobei die Entsicherungsbetätigung eine distale Schubbetätigung umfasst.

16. Die Vorrichtung nach einem der vorhergehenden Ansprüche, weiter umfassend einen Sicherungsverhinderungsmechanismus.

## Revendications

1. Dispositif pour remplacer de façon endovasculaire la valvule cardiaque d'un patient, comprenant:
une gaine (110);
un outil de déploiement (100) comprenant une pluralité d'éléments d'actionnement d'ancrage (106);
une valvule de remplacement (20) configurée pour être disposée à l'intérieur de la gaine (110) pour une distribution dans le voisinage de la valvule cardiaque; et
un ancrage expansible (30) configuré pour être disposé à l'intérieur de la gaine (110) pour une distribution dans le voisinage de la valvule cardiaque, à déployer à partir de la gaine, pour être expansé par l'outil de déploiement afin d'entrer en contact avec un tissu à un site d'ancrage, et pour être récupéré dans la gaine après avoir été expansé,
**caractérisé en ce que** les éléments d'actionnement d'ancrage comprennent une pluralité d'éléments d'actionnement d'ancrage distaux (106b) adaptés pour appliquer une force d'actionnement sur une partie distale de l'ancrage (30), et
**en ce que** les éléments d'actionnement d'ancrage distaux comprennent chacun un premier élément (32) qui est attaché à la partie distale de l'ancrage, et un deuxième élément (34) qui peut être attaché de façon détachable au premier élément.

2. Dispositif selon la revendication 1, dans lequel les premiers éléments comprennent chacun un élément de verrou qui est adapté pour verrouiller l'ancrage dans un état déployé, et comprenant en outre un actionneur de verrou qui est adapté pour appliquer une force de verrouillage afin de verrouiller l'ancrage dans l'état déployé.

3. Dispositif selon la revendication 1 ou 2, comprenant en outre un actionneur de déverrouillage qui est adapté pour déverrouiller l'ancrage.

4. Dispositif selon l'une quelconque des revendications précédentes, dans lequel les éléments d'actionnement d'ancrage comprennent une pluralité d'éléments d'actionnement d'ancrage proximaux adaptés pour appliquer une force d'actionnement à une partie proximale de l'ancrage.

5. Dispositif selon l'une quelconque des revendications précédentes, dans lequel les premiers éléments comprennent chacun une tige adaptée pour supporter la valvule à l'intérieur de l'ancrage.

6. Dispositif selon la revendication 5, dans lequel ladite au moins une tige comprend une structure de fixation de valvule, la valvule de remplacement étant attachée à la tige par l'intermédiaire de la structure de fixation de valvule.

7. Dispositif selon l'une quelconque des revendications précédentes, dans lequel l'élément de verrouillage est en outre adapté pour engager un élément d'ancrage afin de verrouiller l'ancrage dans l'état déployé.

8. Dispositif selon l'une quelconque des revendications précédentes, dans lequel le dispositif comprend en outre des éléments de verrouillage d'ancrage qui sont attachés à l'ancrage et qui sont adaptés pour se coupler à l'élément de verrouillage de chaque premier élément afin de verrouillage l'ancrage dans la configuration déployée.

9. Dispositif selon la revendication 8, dans lequel les éléments de verrouillage d'ancrage comprennent chacun une surface de mise en prise adaptée pour engager le premier élément et pour déplacer au moins une partie de l'élément de verrouillage d'ancrage en réponse à une force d'actionnement de verrou.

10. Dispositif selon la revendication 8, dans lequel au moins un élément de verrouillage de premier élément et au moins un élément de verrouillage d'ancrage sont adaptés pour se coupler dans une pluralité de configurations de couplage.

11. Dispositif selon la revendication 10, dans lequel ledit au moins un élément de verrouillage de premier élément comprend une pluralité de cliquets adaptés pour engager une partie dentée dudit au moins un élément de verrouillage d'ancrage.

12. Dispositif selon l'une quelconque des revendications précédentes, dans lequel les éléments de verrouillage d'ancrage comprennent chacun une boucle.

13. Dispositif selon l'une quelconque des revendications précédentes, dans lequel l'élément de verrouillage de premier élément comprend en outre une caractéristique d'alignement de verrou qui est adaptée pour aligner l'élément de verrouillage de premier élément avec l'élément de verrouillage d'ancrage.

14. Dispositif selon l'une quelconque des revendications précédentes, dans lequel l'élément d'actionnement d'ancrage distal comprend l'actionneur de verrou.

15. Dispositif selon la revendication 3, dans lequel l'actionneur de déverrouillage comprend un actionneur de traction proximal, ou dans lequel l'actionneur de déverrouillage comprend un actionneur de poussée distal.

16. Dispositif selon l'une quelconque des revendications précédentes, comprenant en outre un mécanisme de prévention de verrouillage.
